# EUROPEAN PATENT APPLICATION

(11) **EP 3 647 325 A1**
(43) Date of publication of application: **06.05.2020**
(21) Application number: 18823350.6
(22) Date of filing: 29.06.2018
(51) Int. Cl.: C07K 16/40, A61K 39/395, A61P 3/06

(54) **PHARMACEUTICAL COMPOSITION COMPRISING PCSK-9 ANTIBODY AND USE THEREOF**

(30) Priority: 30.06.2017 CN 201710519829
(71) Applicant: Shanghai Hengrui Pharmaceutical Co., Ltd, Shanghai 200245 (CN); Shanghai Hengrui Pharmaceutical Co., Ltd, Shanghai 200245 (CN)
(72) Inventor: TIAN, Chenmin, Shanghai 200245 (CN); LI, Hao, Shanghai 200245 (CN); LIU, Xun, Shanghai 200245 (CN)
(74) Representative: dompatent von Kreisler Selting Werner - Partnerschaft von Patent- und Rechtsanwälten mbB
(86) International application number: PCT/CN2018/093573
(87) International publication number: WO 2019/001560

(57) **Abstract**

Provided is a pharmaceutical composition comprising a PCSK9 antibody or an antigen-binding fragment thereof in a histidine buffer. In addition, the pharmaceutical composition may also comprise saccharides and nonionic surfactants.

SEQUENCE LISTING

<110>  JIANGSU HENGRUI MEDICINE CO.,LTD; SHANGHAI HENGRUI
       PHARMACEUTICAL CO.,LTD

<120>  Pharmaceutical composition comprising PCSK-9 antibody and use
       thereof

<130>  200105ep

<140>  PCT/CN2018/093573
<141>  2018-06-29

<150>  CN201710519829.6
<151>  2017-06-30

<160>  33

<170>  PatentIn version 3.5

<210>  1
<211>  698
<212>  PRT
<213>  Artificial Sequence

<220>
<223>  PCSK9 with a His tag: PCSK9-His6, used as an immunogen for
       immunizing mice or used as a test reagent

<400>  1

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln His His His His His His
    690                 695

<210>  2
<211>  714
<212>  PRT
<213>  Artificial Sequence

<220>
<223>  PCSK9 with a PADRE peptide and a His-tag: PCSK9-PADRE-His6, used
       as an immunogen, wherein the contained PADRE peptide can promote
       immunization

<400>  2

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu

130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln Gly Ser Gly Ala Lys Phe Val Ala Ala Trp Thr Leu
    690                 695                 700

Lys Ala Ala Ala His His His His His His
705                 710

<210>  3
<211>  704
<212>  PRT
<213>  Artificial Sequence

<220>
<223>  A fusion protein of PCSK9 with a TEV cleavage site and a His tag:
       PCSK9-TEV-His6, N-PCSK9 (N terminal PCSK9 domain), used as an
       immunogen, which can be obtained by TEV cleavage

<400>  3

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly

370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Glu Asn Leu Tyr Phe Gln Gly Ala Gly Trp Gln Leu Phe Cys Arg
    450                 455                 460

Thr Val Trp Ser Ala His Ser Gly Pro Thr Arg Met Ala Thr Ala Val
465                 470                 475                 480

Ala Arg Cys Ala Pro Asp Glu Glu Leu Leu Ser Cys Ser Ser Phe Ser
                485                 490                 495

Arg Ser Gly Lys Arg Arg Gly Glu Arg Met Glu Ala Gln Gly Gly Lys
            500                 505                 510

Leu Val Cys Arg Ala His Asn Ala Phe Gly Gly Glu Gly Val Tyr Ala
        515                 520                 525

Ile Ala Arg Cys Cys Leu Leu Pro Gln Ala Asn Cys Ser Val His Thr
    530                 535                 540

Ala Pro Pro Ala Glu Ala Ser Met Gly Thr Arg Val His Cys His Gln
545                 550                 555                 560

Gln Gly His Val Leu Thr Gly Cys Ser Ser His Trp Glu Val Glu Asp
                565                 570                 575

Leu Gly Thr His Lys Pro Pro Val Leu Arg Pro Arg Gly Gln Pro Asn
            580                 585                 590

Gln Cys Val Gly His Arg Glu Ala Ser Ile His Ala Ser Cys Cys His
        595                 600                 605

Ala Pro Gly Leu Glu Cys Lys Val Lys Glu His Gly Ile Pro Ala Pro
    610                 615                 620

Gln Glu Gln Val Thr Val Ala Cys Glu Glu Gly Trp Thr Leu Thr Gly
625                 630                 635                 640

Cys Ser Ala Leu Pro Gly Thr Ser His Val Leu Gly Ala Tyr Ala Val
                645                 650                 655

Asp Asn Thr Cys Val Val Arg Ser Arg Asp Val Ser Thr Thr Gly Ser
            660                 665                 670

Thr Ser Glu Gly Ala Val Thr Ala Val Ala Ile Cys Cys Arg Ser Arg
        675                 680                 685

His Leu Ala Gln Ala Ser Gln Glu Leu Gln His His His His His His
    690                 695                 700

<210>  4
<211>  698
<212>  PRT
<213>  Artificial Sequence

<220>
<223>  PCSK9-D374Y mutant protein, with a His-tag: PCSK9-D374Y-His6,
       used as a test reagent

<400>  4

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Tyr Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln His His His His His His
    690                 695

<210>  5
<211>  719
<212>  PRT
<213>  Artificial Sequence

<220>
<223>  PCSK9 protein inserted with a biotin receiving peptide BP15 and a
       His tag

<400>  5

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu

385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln Gly Ser Thr Ser Gly Ser Gly Leu Asn Asp Ile Phe
    690                 695                 700

Glu Ala Gln Lys Ile Glu Trp His Glu His His His His His His
705                 710                 715

<210>  6
<211>  719
<212>  PRT
<213>  Artificial Sequence

<220>
<223>  PCSK9 D374Y mutant protein inserted with a biotin receiving
       peptide BP15 and a His tag: PCSK9-D374Y-BP15-His6, as a test
       protein

<400>  6

Met Gly Thr Val Ser Ser Arg Arg Ser Trp Trp Pro Leu Pro Leu Leu
1               5                   10                  15

Leu Leu Leu Leu Leu Leu Leu Gly Pro Ala Gly Ala Arg Ala Gln Glu
            20                  25                  30

Asp Glu Asp Gly Asp Tyr Glu Glu Leu Val Leu Ala Leu Arg Ser Glu
        35                  40                  45

Glu Asp Gly Leu Ala Glu Ala Pro Glu His Gly Thr Thr Ala Thr Phe
    50                  55                  60

His Arg Cys Ala Lys Asp Pro Trp Arg Leu Pro Gly Thr Tyr Val Val
65                  70                  75                  80

Val Leu Lys Glu Glu Thr His Leu Ser Gln Ser Glu Arg Thr Ala Arg
                85                  90                  95

Arg Leu Gln Ala Gln Ala Ala Arg Arg Gly Tyr Leu Thr Lys Ile Leu
            100                 105                 110

His Val Phe His Gly Leu Leu Pro Gly Phe Leu Val Lys Met Ser Gly
        115                 120                 125

Asp Leu Leu Glu Leu Ala Leu Lys Leu Pro His Val Asp Tyr Ile Glu
    130                 135                 140

Glu Asp Ser Ser Val Phe Ala Gln Ser Ile Pro Trp Asn Leu Glu Arg
145                 150                 155                 160

Ile Thr Pro Pro Arg Tyr Arg Ala Asp Glu Tyr Gln Pro Pro Asp Gly
                165                 170                 175

Gly Ser Leu Val Glu Val Tyr Leu Leu Asp Thr Ser Ile Gln Ser Asp
            180                 185                 190

His Arg Glu Ile Glu Gly Arg Val Met Val Thr Asp Phe Glu Asn Val
        195                 200                 205

Pro Glu Glu Asp Gly Thr Arg Phe His Arg Gln Ala Ser Lys Cys Asp
    210                 215                 220

Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
                245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
        275                 280                 285

Leu Ala Gly Gly Tyr Ser Arg Val Leu Asn Ala Ala Cys Gln Arg Leu
    290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
                325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
        355                 360                 365

Ile Gly Ala Ser Ser Tyr Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
                405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
        435                 440                 445

His Gly Ala Gly Trp Gln Leu Phe Cys Arg Thr Val Trp Ser Ala His
    450                 455                 460

Ser Gly Pro Thr Arg Met Ala Thr Ala Val Ala Arg Cys Ala Pro Asp
465                 470                 475                 480

Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
        515                 520                 525

Leu Pro Gln Ala Asn Cys Ser Val His Thr Ala Pro Pro Ala Glu Ala
    530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
        595                 600                 605

Lys Val Lys Glu His Gly Ile Pro Ala Pro Gln Glu Gln Val Thr Val
    610                 615                 620

Ala Cys Glu Glu Gly Trp Thr Leu Thr Gly Cys Ser Ala Leu Pro Gly

625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
        675                 680                 685

Gln Glu Leu Gln Gly Ser Thr Ser Gly Ser Gly Leu Asn Asp Ile Phe
    690                 695                 700

Glu Ala Gln Lys Ile Glu Trp His Glu His His His His His His
705                 710                 715

<210>  7
<211>  802
<212>  PRT
<213>  Artificial Sequence

<220>
<223>  PCSK9 receptor protein LDLR extracellular domain with a Flag tag
       and a His tag: LDLR-ECD-Flag-His6 as a test reagent

<400>  7

Met Gly Pro Trp Gly Trp Lys Leu Arg Trp Thr Val Ala Leu Leu Leu
1               5                   10                  15

Ala Ala Ala Gly Thr Ala Val Gly Asp Arg Cys Glu Arg Asn Glu Phe
            20                  25                  30

Gln Cys Gln Asp Gly Lys Cys Ile Ser Tyr Lys Trp Val Cys Asp Gly
        35                  40                  45

Ser Ala Glu Cys Gln Asp Gly Ser Asp Glu Ser Gln Glu Thr Cys Leu
    50                  55                  60

Ser Val Thr Cys Lys Ser Gly Asp Phe Ser Cys Gly Gly Arg Val Asn
65                  70                  75                  80

Arg Cys Ile Pro Gln Phe Trp Arg Cys Asp Gly Gln Val Asp Cys Asp
                85                  90                  95

Asn Gly Ser Asp Glu Gln Gly Cys Pro Pro Lys Thr Cys Ser Gln Asp
            100                 105                 110

Glu Phe Arg Cys His Asp Gly Lys Cys Ile Ser Arg Gln Phe Val Cys

        115                 120                 125

Asp Ser Asp Arg Asp Cys Leu Asp Gly Ser Asp Glu Ala Ser Cys Pro
    130                 135                 140

Val Leu Thr Cys Gly Pro Ala Ser Phe Gln Cys Asn Ser Ser Thr Cys
145                 150                 155                 160

Ile Pro Gln Leu Trp Ala Cys Asp Asn Asp Pro Asp Cys Glu Asp Gly
                165                 170                 175

Ser Asp Glu Trp Pro Gln Arg Cys Arg Gly Leu Tyr Val Phe Gln Gly
            180                 185                 190

Asp Ser Ser Pro Cys Ser Ala Phe Glu Phe His Cys Leu Ser Gly Glu
        195                 200                 205

Cys Ile His Ser Ser Trp Arg Cys Asp Gly Gly Pro Asp Cys Lys Asp
    210                 215                 220

Lys Ser Asp Glu Glu Asn Cys Ala Val Ala Thr Cys Arg Pro Asp Glu
225                 230                 235                 240

Phe Gln Cys Ser Asp Gly Asn Cys Ile His Gly Ser Arg Gln Cys Asp
                245                 250                 255

Arg Glu Tyr Asp Cys Lys Asp Met Ser Asp Glu Val Gly Cys Val Asn
            260                 265                 270

Val Thr Leu Cys Glu Gly Pro Asn Lys Phe Lys Cys His Ser Gly Glu
        275                 280                 285

Cys Ile Thr Leu Asp Lys Val Cys Asn Met Ala Arg Asp Cys Arg Asp
    290                 295                 300

Trp Ser Asp Glu Pro Ile Lys Glu Cys Gly Thr Asn Glu Cys Leu Asp
305                 310                 315                 320

Asn Asn Gly Gly Cys Ser His Val Cys Asn Asp Leu Lys Ile Gly Tyr
                325                 330                 335

Glu Cys Leu Cys Pro Asp Gly Phe Gln Leu Val Ala Gln Arg Arg Cys
            340                 345                 350

Glu Asp Ile Asp Glu Cys Gln Asp Pro Asp Thr Cys Ser Gln Leu Cys
        355                 360                 365

Val Asn Leu Glu Gly Gly Tyr Lys Cys Gln Cys Glu Glu Gly Phe Gln
    370                 375                 380

Leu Asp Pro His Thr Lys Ala Cys Lys Ala Val Gly Ser Ile Ala Tyr
385                 390                 395                 400

Leu Phe Phe Thr Asn Arg His Glu Val Arg Lys Met Thr Leu Asp Arg
                405                 410                 415

Ser Glu Tyr Thr Ser Leu Ile Pro Asn Leu Arg Asn Val Val Ala Leu
            420                 425                 430

Asp Thr Glu Val Ala Ser Asn Arg Ile Tyr Trp Ser Asp Leu Ser Gln
        435                 440                 445

Arg Met Ile Cys Ser Thr Gln Leu Asp Arg Ala His Gly Val Ser Ser
    450                 455                 460

Tyr Asp Thr Val Ile Ser Arg Asp Ile Gln Ala Pro Asp Gly Leu Ala
465                 470                 475                 480

Val Asp Trp Ile His Ser Asn Ile Tyr Trp Thr Asp Ser Val Leu Gly
                485                 490                 495

Thr Val Ser Val Ala Asp Thr Lys Gly Val Lys Arg Lys Thr Leu Phe
            500                 505                 510

Arg Glu Asn Gly Ser Lys Pro Arg Ala Ile Val Val Asp Pro Val His
        515                 520                 525

Gly Phe Met Tyr Trp Thr Asp Trp Gly Thr Pro Ala Lys Ile Lys Lys
    530                 535                 540

Gly Gly Leu Asn Gly Val Asp Ile Tyr Ser Leu Val Thr Glu Asn Ile
545                 550                 555                 560

Gln Trp Pro Asn Gly Ile Thr Leu Asp Leu Leu Ser Gly Arg Leu Tyr
                565                 570                 575

Trp Val Asp Ser Lys Leu His Ser Ile Ser Ser Ile Asp Val Asn Gly
            580                 585                 590

Gly Asn Arg Lys Thr Ile Leu Glu Asp Glu Lys Arg Leu Ala His Pro
        595                 600                 605

Phe Ser Leu Ala Val Phe Glu Asp Lys Val Phe Trp Thr Asp Ile Ile
    610                 615                 620

Asn Glu Ala Ile Phe Ser Ala Asn Arg Leu Thr Gly Ser Asp Val Asn
625                 630                 635                 640

Leu Leu Ala Glu Asn Leu Leu Ser Pro Glu Asp Met Val Leu Phe His
                645                 650                 655

Asn Leu Thr Gln Pro Arg Gly Val Asn Trp Cys Glu Arg Thr Thr Leu
            660                 665                 670

Ser Asn Gly Gly Cys Gln Tyr Leu Cys Leu Pro Ala Pro Gln Ile Asn
        675                 680                 685

Pro His Ser Pro Lys Phe Thr Cys Ala Cys Pro Asp Gly Met Leu Leu
    690                 695                 700

Ala Arg Asp Met Arg Ser Cys Leu Thr Glu Ala Glu Ala Ala Val Ala
705                 710                 715                 720

Thr Gln Glu Thr Ser Thr Val Arg Leu Lys Val Ser Ser Thr Ala Val
                725                 730                 735

Arg Thr Gln His Thr Thr Thr Arg Pro Val Pro Asp Thr Ser Arg Leu
            740                 745                 750

Pro Gly Ala Thr Pro Gly Leu Thr Thr Val Glu Ile Val Thr Met Ser
        755                 760                 765

His Gln Ala Leu Gly Asp Val Ala Gly Arg Gly Asn Glu Lys Lys Pro
    770                 775                 780

Ser Ser Val Arg Asp Tyr Lys Asp Asp Asp Asp Lys His His His His
785                 790                 795                 800

His His

<210>  8
<211>  331
<212>  PRT
<213>  Artificial Sequence

<220>
<223>  LCDR-Fc, a fusion protein of truncated LDLR extracellular domain
       with a hIgG1 Fc (with PCSK9 binding activity): LDLR -sECD CFc
       (hIgG1) as a test reagent

<400>  8

Met Glu Phe Gly Leu Ser Trp Leu Phe Leu Val Ala Ile Leu Lys Gly
1               5                   10                  15

Val Gln Cys Gly Thr Asn Glu Cys Leu Asp Asn Asn Gly Gly Cys Ser
            20                  25                  30

His Val Cys Asn Asp Leu Lys Ile Gly Tyr Glu Cys Leu Cys Pro Asp
        35                  40                  45

Gly Phe Gln Leu Val Ala Gln Arg Arg Cys Glu Asp Ile Asp Glu Cys
    50                  55                  60

Gln Asp Pro Asp Thr Cys Ser Gln Leu Cys Val Asn Leu Glu Gly Gly
65                  70                  75                  80

Tyr Lys Cys Gln Cys Glu Glu Gly Phe Gln Leu Asp Pro His Thr Lys
                85                  90                  95

Ala Cys Lys Glu Pro Lys Ser Ser Asp Lys Thr His Thr Cys Pro Pro
            100                 105                 110

Cys Pro Ala Pro Glu Leu Leu Gly Gly Pro Ser Val Phe Leu Phe Pro
        115                 120                 125

Pro Lys Pro Lys Asp Thr Leu Met Ile Ser Arg Thr Pro Glu Val Thr
    130                 135                 140

Cys Val Val Val Asp Val Ser His Glu Asp Pro Glu Val Lys Phe Asn
145                 150                 155                 160

Trp Tyr Val Asp Gly Val Glu Val His Asn Ala Lys Thr Lys Pro Arg
                165                 170                 175

Glu Glu Gln Tyr Asn Ser Thr Tyr Arg Val Val Ser Val Leu Thr Val
            180                 185                 190

Leu His Gln Asp Trp Leu Asn Gly Lys Glu Tyr Lys Cys Lys Val Ser
        195                 200                 205

Asn Lys Ala Leu Pro Ala Pro Ile Glu Lys Thr Ile Ser Lys Ala Lys
    210                 215                 220

Gly Gln Pro Arg Glu Pro Gln Val Tyr Thr Leu Pro Pro Ser Arg Asp
225                 230                 235                 240

Glu Leu Thr Lys Asn Gln Val Ser Leu Thr Cys Leu Val Lys Gly Phe
                245                 250                 255

Tyr Pro Ser Asp Ile Ala Val Glu Trp Glu Ser Asn Gly Gln Pro Glu

260                 265                 270

Asn Asn Tyr Lys Thr Thr Pro Pro Val Leu Asp Ser Asp Gly Ser Phe
        275                 280                 285

Phe Leu Tyr Ser Lys Leu Thr Val Asp Lys Ser Arg Trp Gln Gln Gly
    290                 295                 300

Asn Val Phe Ser Cys Ser Val Met His Glu Ala Leu His Asn His Tyr
305                 310                 315                 320

Thr Gln Lys Ser Leu Ser Leu Ser Pro Gly Lys
                325                 330

<210>  9
<211>  294
<212>  PRT
<213>  Artificial Sequence

<220>
<223>  A fusion protein of a more truncated LDLR extracellular domain
       with a hIgG1 Fc (with PCSK9 binding activity): LDLR-ssECD CFc
       (hIgG1) as a test reagent

<400>  9

Met Glu Phe Gly Leu Ser Trp Leu Phe Leu Val Ala Ile Leu Lys Gly
1               5                   10                  15

Val Gln Cys Gly Thr Asn Glu Cys Leu Asp Asn Asn Gly Gly Cys Ser
            20                  25                  30

His Val Cys Asn Asp Leu Lys Ile Gly Tyr Glu Cys Leu Cys Pro Asp
        35                  40                  45

Gly Phe Gln Leu Val Ala Gln Arg Arg Cys Glu Asp Ile Asp Glu Pro
    50                  55                  60

Lys Ser Ser Asp Lys Thr His Thr Cys Pro Pro Cys Pro Ala Pro Glu
65                  70                  75                  80

Leu Leu Gly Gly Pro Ser Val Phe Leu Phe Pro Pro Lys Pro Lys Asp
                85                  90                  95

Thr Leu Met Ile Ser Arg Thr Pro Glu Val Thr Cys Val Val Val Asp
            100                 105                 110

Val Ser His Glu Asp Pro Glu Val Lys Phe Asn Trp Tyr Val Asp Gly
        115                 120                 125

Val Glu Val His Asn Ala Lys Thr Lys Pro Arg Glu Glu Gln Tyr Asn
    130                 135                 140

Ser Thr Tyr Arg Val Val Ser Val Leu Thr Val Leu His Gln Asp Trp
145                 150                 155                 160

Leu Asn Gly Lys Glu Tyr Lys Cys Lys Val Ser Asn Lys Ala Leu Pro
                165                 170                 175

Ala Pro Ile Glu Lys Thr Ile Ser Lys Ala Lys Gly Gln Pro Arg Glu
            180                 185                 190

Pro Gln Val Tyr Thr Leu Pro Pro Ser Arg Asp Glu Leu Thr Lys Asn
        195                 200                 205

Gln Val Ser Leu Thr Cys Leu Val Lys Gly Phe Tyr Pro Ser Asp Ile
    210                 215                 220

Ala Val Glu Trp Glu Ser Asn Gly Gln Pro Glu Asn Asn Tyr Lys Thr
225                 230                 235                 240

Thr Pro Pro Val Leu Asp Ser Asp Gly Ser Phe Phe Leu Tyr Ser Lys
                245                 250                 255

Leu Thr Val Asp Lys Ser Arg Trp Gln Gln Gly Asn Val Phe Ser Cys
            260                 265                 270

Ser Val Met His Glu Ala Leu His Asn His Tyr Thr Gln Lys Ser Leu
        275                 280                 285

Ser Leu Ser Pro Gly Lys
    290

<210>  10
<211>  121
<212>  PRT
<213>  Mus musculus

<400>  10

Gln Val His Leu Gln Gln Ser Gly Ala Glu Leu Ala Lys Pro Gly Ala
1               5                   10                  15

Ser Val Lys Leu Ser Cys Lys Ala Ser Gly Tyr Thr Phe Asn Asp Tyr
            20                  25                  30

Trp Met His Trp Val Lys Glu Arg Pro Gly Gln Gly Leu Glu Trp Ile
        35                  40                  45

Gly Tyr Ile Asn Pro Ser Ser Gly Phe Thr Lys Tyr His Gln Asn Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Tyr Asp Asp Ser Ala Val Tyr Tyr Cys
                85                  90                  95

Ala Arg Gln Tyr Asp Tyr Asp Glu Asp Trp Tyr Phe Asp Val Trp Gly
            100                 105                 110

Thr Gly Thr Thr Val Thr Val Ser Ser
        115                 120

<210>  11
<211>  112
<212>  PRT
<213>  Mus musculus

<400>  11

Asp Ile Val Met Ser Gln Ser Pro Ser Ser Leu Ala Val Ser Ala Gly
1               5                   10                  15

Glu Lys Val Thr Met Ser Cys Lys Ser Ser Gln Ser Leu Leu Asn Ser
            20                  25                  30

Arg Thr Arg Lys Asn Phe Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Arg Gly Ser Gly Thr Asp Phe Thr Leu Thr
65                  70                  75                  80

Ile Ser Ser Val Gln Ala Glu Asp Leu Ala Val Tyr Tyr Cys Lys Gln
                85                  90                  95

Ser Phe Asn Leu Phe Thr Phe Gly Ser Gly Thr Lys Leu Glu Ile Lys
            100                 105                 110

<210>  12
<211>  5
<212>  PRT
<213>  Mus musculus

<400>  12

Asp Tyr Trp Met His

1                   5


<210>  13
<211>  17
<212>  PRT
<213>  Mus musculus

<400>  13

Tyr Ile Asn Pro Ser Ser Gly Phe Thr Lys Tyr His Gln Asn Phe Lys
1               5                   10                  15


Asp




<210>  14
<211>  12
<212>  PRT
<213>  Mus musculus

<400>  14

Gln Tyr Asp Tyr Asp Glu Asp Trp Tyr Phe Asp Val
1               5                   10


<210>  15
<211>  17
<212>  PRT
<213>  Mus musculus

<400>  15

Lys Ser Ser Gln Ser Leu Leu Asn Ser Arg Thr Arg Lys Asn Phe Leu
1               5                   10                  15


Ala




<210>  16
<211>  7
<212>  PRT
<213>  Mus musculus

<400>  16

Trp Ala Ser Thr Arg Glu Ser
1               5


<210>  17
<211>  8
<212>  PRT
<213>  Mus musculus

<400>  17

Lys Gln Ser Phe Asn Leu Phe Thr

1               5


<210>  18
<211>  121
<212>  PRT
<213>  Artificial Sequence

<220>
<223>  ch-001

<400>  18

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Trp Met His Trp Val Arg Gln Ala Pro Gly Gln Gly Leu Glu Trp Met
        35                  40                  45


Gly Tyr Ile Asn Pro Ser Ser Gly Phe Thr Lys Tyr His Gln Asn Phe
    50                  55                  60


Lys Asp Arg Val Thr Met Thr Arg Asp Thr Ser Ile Ser Thr Ala Tyr
65                  70                  75                  80


Met Glu Leu Ser Arg Leu Arg Ser Asp Asp Thr Ala Val Tyr Tyr Cys
                85                  90                  95


Ala Arg Gln Tyr Asp Tyr Asp Glu Asp Trp Tyr Phe Asp Val Trp Gly
            100                 105                 110


Gln Gly Thr Thr Val Thr Val Ser Ser
        115                 120


<210>  19
<211>  121
<212>  PRT
<213>  Artificial Sequence

<220>
<223>  h001-VH.1A

<400>  19

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Asn Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser
        115                 120

<210>  20
<211>  121
<212>  PRT
<213>  Artificial Sequence

<220>
<223>  h001-VH.1B

<400>  20

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Thr Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser

115                 120

<210>  21
<211>  121
<212>  PRT
<213>  Artificial Sequence

<220>
<223>  h001-VH.1C

<400>  21

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Asn Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Thr Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser
        115                 120

<210>  22
<211>  121
<212>  PRT
<213>  Artificial Sequence

<220>
<223>  h001-VH.1D

<400>  22

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Asn Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Ala Asp Lys Ser Ile Ser Thr Ala Tyr
65                  70                  75                  80

Met Glu Leu Ser Arg Leu Thr Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser
        115                 120

<210>  23
<211>  121
<212>  PRT
<213>  Artificial Sequence

<220>
<223>  h001-VH.1E

<400>  23

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Asn Asp Tyr
            20                  25                  30

Trp Met His Trp Val Lys Gln Ala Pro Gly Gln Gly Leu Glu Trp Ile
        35                  40                  45

Gly Tyr Ile Asn Pro Ser Ser Gly Phe Thr Lys Tyr His Gln Asn Phe
    50                  55                  60

Lys Asp Lys Ala Thr Leu Thr Ala Asp Lys Ser Ile Ser Thr Ala Tyr
65                  70                  75                  80

Met Glu Leu Ser Arg Leu Thr Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser

115                 120

<210>  24
<211>  112
<212>  PRT
<213>  Artificial Sequence

<220>
<223>  ch-001 hVL.1 (CDR grafted)

<400>  24

Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Phe Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Phe Asn Leu Phe Thr Phe Gly Gln Gly Thr Lys Leu Glu Ile Lys
            100                 105                 110

<210>  25
<211>  112
<212>  PRT
<213>  Artificial Sequence

<220>
<223>  h001-VL.1A

<400>  25

Asp Ile Gln Met Thr Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Phe Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Phe Asn Leu Phe Thr Phe Gly Gln Gly Thr Lys Leu Glu Ile Lys
            100                 105                 110

<210>  26
<211>  112
<212>  PRT
<213>  Artificial Sequence

<220>
<223>  h001-VL.1B

<400>  26

Asp Ile Gln Met Ser Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Phe Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Phe Asn Leu Phe Thr Phe Gly Gln Gly Thr Lys Leu Glu Ile Lys
            100                 105                 110

<210>  27
<211>  112
<212>  PRT
<213>  Artificial Sequence

<220>
<223>  h001-VL.1C

<400>  27

Asp Ile Val Met Ser Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Phe Leu Ala Trp Tyr Gln Gln Lys Pro Gly Lys
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Phe Asn Leu Phe Thr Phe Gly Gln Gly Thr Lys Leu Glu Ile Lys
            100                 105                 110

<210>  28
<211>  451
<212>  PRT
<213>  Artificial Sequence

<220>
<223>  h001-4-IgG1

<400>  28

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  29
<211>  1413
<212>  DNA
<213>  Artificial Sequence

<220>
<223>  h001-4-IgG1

<400>  29
atggagtttg ggctgagctg gctttttctt gtcgcgattc ttaagggtgt ccagtgccag      60

gtgcagctgg tgcagagcgg cgctgaggtg aagaagcccg gagcgagcgt aaaggtgagc     120

tgcaaggcca gcggatacac cttcaccgac tactggatgc actgggtgag gcaggcccca     180

ggacagggcc tggagtggat gggctacatc aaccccagca gcggctttac caagtatcac     240

cagaacttca aagacagggt gaccatgacc agggacacca gcatcagcac cgcctacatg     300

gagctgagca ggctgaggag cgacgacacc gccgtgtact actgcgccag gcaatacgac     360

tacgacgagg actggtactt cgacgtgtgg ggccaaggaa ccaccgtgac tgtgagcagc     420

gcttcgacca agggcccatc ggtcttcccc ctggcaccct cctccaagag cacctctggg     480

ggcacagcgg ccctgggctg cctggtcaag gactacttcc ccgaaccggt gacggtgtcg     540

tggaactcag gcgccctgac cagcggcgtg cacaccttcc cggctgtcct acagtcctca     600

ggactctact ccctcagcag cgtggtgacc gtgccctcca gcagcttggg cacccagacc     660

tacatctgca acgtgaatca caagcccagc aacaccaagg tggacaagaa agttgagccc     720

aaatcttgtg acaaaactca cacatgccca ccgtgcccag cacctgaact cctgggggga     780

ccgtcagtct tcctcttccc cccaaaaccc aaggacaccc tcatgatctc ccggacccct 840

gaggtcacat gcgtggtggt ggacgtgagc cacgaagacc ctgaggtcaa gttcaactgg 900

tacgtggacg gcgtggaggt gcataatgcc aagacaaagc cgcgggagga gcagtacaac 960

agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080

aaagccaaag ggcagccccg agaaccacag gtgtacaccc tgcccccatc ccgggatgag 1140

ctgaccaaga accaggtcag cctgacctgc ctggtcaaag gcttctatcc cagcgacatc 1200

gccgtggagt gggagagcaa tgggcagccg gagaacaact acaagaccac gcctcccgtg 1260

ctggactccg acggctcctt cttcctctac agcaagctca ccgtggacaa gagcaggtgg 1320

cagcagggga acgtcttctc atgctccgtg atgcatgagg ctctgcacaa ccactacacg 1380

cagaagagcc tctccctgtc tccgggtaaa tga 1413

<210> 30
<211> 219
<212> PRT
<213> Artificial Sequence

<220>
<223> h001-4-kappa

<400> 30

Asp Ile Val Met Ser Gln Ser Pro Ser Ser Leu Ser Ala Ser Val Gly
1               5                   10                  15

Asp Arg Val Thr Ile Thr Cys Lys Ser Ser Gln Ser Leu Leu Asn Ser
            20                  25                  30

Arg Thr Arg Lys Asn Phe Leu Ala Trp Tyr Gln Gln Lys Pro Gly Lys
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Phe Asn Leu Phe Thr Phe Gly Gln Gly Thr Lys Leu Glu Ile Lys
            100                 105                 110

Arg Thr Val Ala Ala Pro Ser Val Phe Ile Phe Pro Pro Ser Asp Glu

115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  31
<211>  726
<212>  DNA
<213>  Artificial Sequence

<220>
<223>  h001-4-kappa

<400>  31
atggacatgc gcgtgcccgc ccagctgctg ggcctgctgc tgctgtggtt ccccggctcg      60

cgatgcgaca tcgtgatgtc tcagagccca tctagcctga gcgccagcgt gggcgacagg     120

gtaaccatca cctgcaagag cagccaaagc ctgctgaaca gcaggacccg caagaacttc     180

ctggcttggt atcagcagaa gcccggcaag tctcccaagt tgctgatcta ctgggccagc     240

accagggaga gcggcgtgcc cgacaggttc agcggctccg gcagcggcac cgacttcacc     300

ctgaccatct ctagtctgca gcccgaggac ttcgccacct actactgcaa gcagagcttc     360

aatctgttca ccttcggcca gggcaccaag ctggagatca agcgtacggt ggctgcacca     420

tctgtcttca tcttcccgcc atctgatgag cagttgaaat ctggaactgc ctctgttgtg     480

tgcctgctga ataacttcta tcccagagag gccaaagtac agtggaaggt ggataacgcc     540

ctccaatcgg gtaactccca ggagagtgtc acagagcagg acagcaagga cagcacctac     600

agcctcagca gcaccctgac gctgagcaaa gcagactacg agaaacacaa agtctacgcc     660

tgcgaagtca cccatcaggg cctgagctcg cccgtcacaa agagcttcaa caggggagag     720

tgttga                                                                726

<210>  32
<211>  451
<212>  PRT
<213>  Artificial Sequence

<220>
<223>  h001-4-IgG1-YTE heavy chain

<400>  32

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Trp Met His Trp Val Arg Gln Ala Pro Gly Gln Gly Leu Glu Trp Met
        35                  40                  45

Gly Tyr Ile Asn Pro Ser Ser Gly Phe Thr Lys Tyr His Gln Asn Phe
    50                  55                  60

Lys Asp Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gln Tyr Asp Tyr Asp Glu Asp Trp Tyr Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys

210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Tyr
                245                 250                 255

Ile Thr Arg Glu Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  33
<211>  1413
<212>  DNA
<213>  Artificial Sequence

<220>
<223>  h001-4-IgG1-YTE heavy chain

<400>  33
atggagtttg ggctgagctg gctttttctt gtcgcgattc ttaagggtgt ccagtgccag        60

gtgcagctgg tgcagagcgg cgctgaggtg aagaagcccg gagcgagcgt aaaggtgagc       120

tgcaaggcca gcggatacac cttcaccgac tactggatgc actgggtgag gcaggcccca       180

ggacagggcc tggagtggat gggctacatc aaccccagca gcggctttac caagtatcac       240

cagaacttca aagacagggt gaccatgacc agggacacca gcatcagcac cgcctacatg       300

gagctgagca ggctgaggag cgacgacacc gccgtgtact actgcgccag gcaatacgac       360

tacgacgagg actggtactt cgacgtgtgg ggccaaggaa ccaccgtgac tgtgagcagc       420

gcttcgacca agggcccatc ggtcttcccc ctggcaccct cctccaagag cacctctggg       480

ggcacagcgg ccctgggctg cctggtcaag gactacttcc ccgaaccggt gacggtgtcg       540

tggaactcag gcgccctgac cagcggcgtg cacaccttcc cggctgtcct acagtcctca       600

ggactctact ccctcagcag cgtggtgacc gtgccctcca gcagcttggg cacccagacc       660

tacatctgca acgtgaatca caagcccagc aacaccaagg tggacaagaa agttgagccc       720

aaatcttgtg acaaaactca cacatgccca ccgtgcccag cacctgaact cctgggggga       780

ccgtcagtct tcctcttccc cccaaaaccc aaggacaccc tctacatcac ccgggagcct       840

gaggtcacat gcgtggtggt ggacgtgagc cacgaagacc ctgaggtcaa gttcaactgg       900

tacgtggacg gcgtggaggt gcataatgcc aagacaaagc cgcgggagga gcagtacaac       960

agcacgtacc gtgtggtcag cgtcctcacc gtcctgcacc aggactggct gaatggcaag      1020

gagtacaagt gcaaggtctc caacaaagcc ctcccagccc ccatcgagaa aaccatctcc      1080

aaagccaaag ggcagccccg agaaccacag gtgtacaccc tgcccccatc ccgggatgag      1140

ctgaccaaga accaggtcag cctgacctgc ctggtcaaag gcttctatcc cagcgacatc      1200

gccgtggagt gggagagcaa tgggcagccg gagaacaact acaagaccac gcctcccgtg      1260

ctggactccg acggctcctt cttcctctac agcaagctca ccgtggacaa gagcaggtgg      1320

cagcagggga acgtcttctc atgctccgtg atgcatgagg ctctgcacaa ccactacacg      1380

cagaagagcc tctccctgtc tccgggtaaa tga                                   1413

## Description

This application claims priority of Chinese Patent Application No. CN201710519829.6, filed on June 30, 2017. The entire content of the aforementioned application is hereby incorporated by reference.

### FIELD OF THE INVENTION

The invention belongs to the field of pharmaceutical formulation, in particular relates to a pharmaceutical composition comprising an anti-PCSK-9 antibody and an antigen-binding fragment thereof, and the use thereof as a medicament.

### BACKGROUND OF THE INVENTION

Hypercholesterolemia is a disease with abnormal metabolism of lipid, characterized by an increased level of serum cholesterol. Its main manifestation is the increased level of serum cholesterol, which causes cholesterol aggregation in blood vessels and consequently forms atherosclerosis. Abundant clinical and experimental research results have proven that the abnormal metabolism of lipid is closely correlated with the occurrence and development of coronary heart disease. Therefore, reducing the concentration of cholesterol in blood has become a major means for treating and preventing atherosclerosis.

With the rapid improvement of the standard of living, dyslipidemia has also become a major factor that endangers urban and rural residents in China. According to the statistics of 2012, about 40% of deaths per year in China were attributed to cardiovascular diseases. At present, the morbidity of dyslipidemia among adults in China is 18.6%, with an estimated 160 million people suffering from dyslipidemia. The morbidities of different types of dyslipidemia are as follows: 2.9% for hypercholesterolemia, 11.9% for hypertriglyceridemia, 7.4% for low high density lipoproteinemia, and 3.9% for marginally increased blood cholesterol level. In the "Chinese Experts Consensus on Prevention and Control of Chronic Disease" reached by the Branch Committee of Chronic Disease Prevention and Control, the Committee of Experts on Disease Prevention and Control, Ministry of Public Health, 2012, it was mentioned that there are 33 million of people suffering from hypercholesterolemia in China, however, from the perspective of local areas, the morbidity of dyslipidemia is far more serious than the above data.

At present, the medicaments clinically used for controlling lipid levels are mainly focused on statins. Lipitor®, as the most widely used and the best-selling cholesterol-lowering medicament, reduces the production of cholesterol by blocking the effect of a cholesterol-producing enzyme in liver, and increases the uptake of cholesterol from blood by the liver, thereby reducing the concentration of cholesterol in blood. However, Lipitor® has its disadvantages. Firstly, it will be understood from data that Lipitor® can reduce low density lipoprotein by 30% to 40%, however, an effectively reduced blood lipid level still cannot be achieved in many patients (the concentration of low density lipoprotein < 50mg/dL). Secondly, there is a difference in response rate to Lipitor® among patients of different races. For these reasons, patients still need a more effective medicine to reduce blood lipid.

As an autosomal dominant monogenic hereditary disease, familial hypercholesterolemia (FM) is clinically characterized by significant increases in total cholesterol (TC) and low density lipoprotein-cholesterol (LDL-c) in blood, xanthelasmata, corneal arcus and premature cardiovascular disease. Mutations in low density lipoprotein receptor (LDL receptor, LDLR) gene causes LDLR deficiency or absence. Consequently, LDL-c will not be transported to the liver for degradation, resulting in an increased level of LDL-c in blood. Currently, three genes, LDLR gene, apolipoprotein B100 gene and proprotein convertase subtilisin/kexin type 9 (PCSK9) gene, have been identified to be correlated with the occurrence of FM.

As a proprotein convertase, proprotein convertase subtilisin/kexin type 9 (PCSK9) belongs to a subfamily of protease K in the secretory *Bacillus subtilis* family. The encoded protein is synthesized as a soluble proenzyme, and intramolecularly processed in the endoplasmic reticulum by self-catalyzing. According to experimental results, PCSK9 can promote the degradation of LDL receptor and thus increases the content of LDL cholesterol in plasma. LDL receptor mediates the endocytosis process of LDL in liver, which is a main pathway to remove LDL from the circulating system. It has been found that PCSK9 gene mutations were identified in 12.5% of hypercholesterolemia (ADH) patients. There are various types of PCSK9 mutations. According to different impacts of mutations on the LDL-c level regulated by PCSK9, the mutations can be divided into two types, loss-of-function type and gain-of-function type. Among them, loss-of-function mutations are associated with low blood cholesterol level and prevent the occurrence of atherosclerotic heart disease. The mutation rate of PCSK9 associated with low cholesterol is higher in African populations than in other races. Gain-of-function mutations of PCSK9 increase plasma cholesterol level by increasing PCSK9 function and reducing LDLR expression, which can lead to severe hypercholesterolemia and premature coronary atherosclerotic heart disease. At present, it is found that gain-of-function mutations of PCSK9 include D374Y, S127R, F216L, N157K, R306S, and so on. In comparison with the PCSK9 wild type, the LDLR on the cell surface of D374Y mutants was decreased by 36%, and that of S127R mutant was decreased by 10%.

However, antibodies become unstable because of their large molecular weights, complicated structures, and susceptibility to degradation, polymerization, or undesirable chemical modification. In order to make antibodies that are suitable for administration, maintain their stability during storage and subsequent use, and exert better effects, research on stable formulations of antibodies is particularly important.

Several companies are currently developing anti-PCSK-9 antibodies and pharmaceutical formulations, such as those of CN103717237A, CN104364266A etc. However, for the new anti-PCSK-9 antibodies, there is still a need to develop a pharmaceutical (formulation) composition comprising an anti-PCSK-9 antibody that is more suitable for administration.

### DETAILED DESCRIPTION OF THE INVENTION

The present invention provides a pharmaceutical composition, comprising an anti-PCSK9 antibody or an antigen binding fragment thereof, and a buffer, wherein the buffer is preferably a histidine buffer or succinate buffer or phosphate buffer or citrate buffer, more preferably a histidine buffer, most preferably a histidine-hydrochloride buffer.

In an alternative embodiment, wherein the concentration of the anti-PCSK-9 antibody or the antigen-binding fragment thereof in the pharmaceutical composition is about 1mg/ml to 150mg/ml, preferably 30mg/ml to 100mg/ml, more preferably 50mg/ml to 60mg/ml, most preferably 50 mg/ml. Non-limiting examples of such concentrations of the anti-PCSK-9 antibody or the antigen-binding fragment thereof include 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, or 60 mg/ml.

In an alternative embodiment, the concentration of the buffer is about 5mM to 50mM, preferably 5mM to 30mM, non-limiting examples of concentrations of the buffer include 10 mM, 12 mM, 14 mM, 16 mM, 18 mM, 20 mM, 22 mM, 24 mM, 26 mM, 28 mM and 30 mM, more preferably 10mM to 15mM, most preferably 10mM.

In an alternative embodiment, the pH of the buffer in the pharmaceutical composition is about 5.5 to 6.5, preferably about 6.0 to 6.5, non-limiting examples of pH of the buffer include about 6.0, about 6.1, about 6.2, about 6.3, about 6.4, or about 6.5.

Further, in an alternative embodiment, the pharmaceutical composition further comprises a saccharide. The "saccharide" herein comprises the conventional composition (CH₂O)n and derivatives thereof, including monosaccharides, disaccharides, trisaccharides, polysaccharides, sugar alcohols, reducing sugars, non-reducing sugars, etc. Examples of saccharides herein include glucose, sucrose, trehalose, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, sorbitol, maltitol, lactitol, iso-maltulose, etc. The preferred saccharide herein is a non-reducing disaccharide, more preferably trehalose or sucrose.

In an alternative embodiment, wherein the concentration of the saccharide in the pharmaceutical is about 10mg/ml to 75mg/ml, preferably 20mg/ml to 60mg/ml, more preferably about 20mg/ml to 40mg/ml, most preferably 25mg/ml. Non-limiting examples of the concentration of the saccharide include 20 mg/ml, 21 mg/ml, 22 mg/ml, 23 mg/ml, 23 mg/ml, 24 mg/ml, 25 mg/ml, 26 mg/ml, 27 mg/ml, 28 mg/ml, 29 mg/ml. 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, or 40 mg/ml.

In an alternative embodiment, the pharmaceutical composition further comprises a surfactant. Herein, the "surfactant" could be selected from the group consisting of a polysorbate 20, polysorbate 80, poloxamer, Triton, sodium dodecyl sulfate, sodium laurel sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl- sarcosine, stearyl-sarcosine, linoleyl- betaine, myristyl- betaine, cetyl-betaine, lauroamidopropyl-betaine, cocamidopropyl-betaine, linoleamidopropyl-betaine, myristamidopropyl-betaine, palmidopropyl- betaine, isostearamidopropyl-betaine, myristamidopropyl-dimethylamine, palmidopropyl-dimethylamine, isostearamidopropyl-dimethylamine, sodium methyl cocoyltaurate, sodium methyl oleyl-taurate, polyethyl glycol, polypropyl glycol, and copolymers of ethylene and propylene glycol etc. The preferred surfactant herein is polysorbate 20 or polysorbate 80, more preferably polysorbate 80.

In an alternative embodiment, the concentration of the surfactant in the pharmaceutical composition is about 0.05mg/ml to 1.0mg/ml, preferably 0.1mg/ml to 0.4mg/ml, non-limiting examples of concentration of the surfactant include 0.1 mg/ml, 0.15 mg/ml, 0.2 mg/ml, 0.25 mg/ml, 0.3 mg/ml, 0.35 mg/ml, 0.4 mg/ml, further preferably 0.1mg/ml to 0.3mg/ml, more preferably 0.1mg/ml to 0.2mg/ml, most preferably 0.2mg/ml.

In an alternative embodiment, the pharmaceutical composition comprises:
(a) 1-150 mg/ml anti-PCSK-9 antibody or antigen-binding fragment thereof,
(b) 5-30 mM histidine buffer, pH about 5.5-6.5, more preferably about 6.0-6.5,
(c) 10-75 mg/ml sucrose, and
(d) 0.05-0.6 mg/ml polysorbate 80.

In an alternative embodiment, the pharmaceutical composition comprises:
(a) 50 mg/ml anti-PCSK-9 antibody or antigen-binding fragment thereof;
(b) 5-20 mM histidine buffer;
(c) 25 mg/ml sucrose; and
(d) 0.1-0.3 mg/ml polysorbate 80.

In an alternative embodiment, the pharmaceutical composition comprises:
(a) 1-150 mg/ml anti-PCSK-9 antibody or antigen-binding fragment thereof, and
(b) 5-30 mM histidine buffer; and the pH of the pharmaceutical composition is about 6.0-6.5.

In an alternative embodiment, the pharmaceutical composition comprises:
(a) 1-150 mg/ml anti-PCSK-9 antibody or antigen-binding fragment thereof;
(b) 5-30 mM histidine buffer;
(c) 10-75 mg/ml sucrose; and
(d) 0.05-0.6 mg/ml polysorbate 80, and the pH of the pharmaceutical composition is about 6.0-6.5, and the histidine buffer is preferably a histidine-hydrochloride buffer.

In an alternative embodiment, the pharmaceutical composition comprises:
(a) 50 mg/ml anti-PCSK-9 antibody or antigen-binding fragment thereof;
(b) 5-20 mM histidine buffer;
(c) 25 mg/ml of sucrose; and
(d) 0.1-0.3 mg/ml polysorbate 80, and the pH of the pharmaceutical composition is about 6.0-6.5, and the histidine buffer is preferably a histidine-hydrochloride buffer.

In an alternative embodiment, the pharmaceutical composition comprises:
(a) 50 mg/ml anti-PCSK-9 antibody or antigen-binding fragment thereof;
(b) 10 mM histidine buffer;
(c) 25 mg/ml sucrose; and
(d) 0.2 mg/ml polysorbate 80, and the pH of the pharmaceutical composition is 6.3 ± 0.1, and the histidine buffer is preferably a histidine-hydrochloride buffer.

In an alternative embodiment, the pharmaceutical composition comprises:
(a) 150 mg/ml anti-PCSK-9 antibody or antigen-binding fragment thereof;
(b) 30 mM histidine-hydrochloride buffer;
(c) 75 mg/ml sucrose; and
(d) 0.6 mg/ml polysorbate 80; the final pH of the pharmaceutical composition is 6.3.

In an alternative embodiment, the pharmaceutical composition comprises:
(a) 50 mg/ml anti-PCSK-9 antibody or antigen-binding fragment thereof;
(b) 10 mM histidine-hydrochloride buffer, pH 6.0;
(c) 25 mg/ml sucrose; and
(d) 0.2 mg/ml polysorbate 80.

In an alternative embodiment, the pharmaceutical composition comprises:
(a) 150 mg/ml anti-PCSK-9 antibody or antigen-binding fragment thereof;
(b) 20 mM histidine-hydrochloride buffer, pH 6.5; and
(c) 70 mg/ml sucrose.

In an alternative embodiment, the pharmaceutical composition comprises:
(a) 150 mg/ml anti-PCSK-9 antibody or antigen-binding fragment thereof;
(b) 20 mM histidine-hydrochloride buffer, pH 6.5; and
(c) 70 mg/ml of α, α-dihydratetrehalose.

In an alternative embodiment, the pharmaceutical composition comprises:
(a) 50 mg/ml anti-PCSK-9 antibody or antigen-binding fragment thereof;
(b) 20 mM histidine-hydrochloride buffer, pH 6.0;
(c) 25 mg/ml sucrose; and
(d) 0.2 mg /ml polysorbate 80.

In an alternative embodiment, the pharmaceutical composition comprises:
(a) 50 mg/ml anti-PCSK-9 antibody or antigen-binding fragment thereof;
(b) 20 mM histidine-hydrochloride buffer, pH 6.5;
(c) 25 mg/ml sucrose; and
(d) 0.2 mg /ml polysorbate 80.

In an alternative embodiment, the anti-PCSK-9 antibody or the antigen binding fragment thereof in the pharmaceutical composition comprises HCDR1, HCDR2 and HCR3 having the sequences of SEQ ID NO: 12, SEQ ID NO: 13 and SEQ ID NO: 14, respectively, and
LCDR1, LCDR2 and LCDR3 having the sequences of SEQ ID NO: 15, SEQ ID NO: 16, and SEQ ID NO: 17, respectively.

In an alternative embodiment, the anti-PCSK-9 antibody or the antigen binding fragment thereof in the pharmaceutical composition is selected from the group consisting of a murine antibody, a chimeric antibody, and a humanized antibody, preferably a humanized antibody.

In an alternative embodiment, the light chain of the anti-PCSK-9 antibody in the pharmaceutical composition has at least 85%, 86%, 87%, 88%, 89%, 90%, 91%, 92%, 93%, 94%, 95%, 96%, 97%, 98%, 99% or 100% sequence identity to the amino acid sequence of the light chain of h001-4-YTE antibody, wherein the heavy chain of the anti-PCSK-9 antibody in the pharmaceutical composition has at least 85%, 86%, 87%, 88%, 89%, 90%, 91%, 92%, 93%, 94%, 95%, 96%, 97%, 98%, 99% or 100% sequence identity to the amino acid sequence of the heavy chain of h001-4-YTE antibody. The light chain sequence of h001-4-YTE antibody has the amino acid sequence of SEQ ID NO: 30, and the heavy chain sequence of h001-4-YTE antibody has the amino acid sequence of SEQ ID NO: 32.

The present invention further provides a method for preparing the pharmaceutical composition described above, comprising the step of replacing the stock solution of an anti-PCSK-9 antibody or an antigen-binding fragment thereof with a buffer. Preferably, the buffer is a histidine buffer, more preferably a histidine hydrochloride buffer. The concentration of the buffer is preferably about 5 mM to 30 mM, and non-limiting examples of concentration of the buffer include 5mM, 6mM, 7mM, 8mM, 9mM, 10mM, 12mM, 14mM, 16mM, 18mM, 20mM, 22mM, 24mM, 26mM, 28mM, and 30mM, more preferably 10mM to 15mM; wherein the pH of the buffer is about 6.0 to 6.5, non-limiting examples include 6.0, 6.1, 6.2, 6.3, 6.4, and 6.5.

Further, the method for preparing the pharmaceutical composition described above further comprises the step of adding sucrose and polysorbate 80 to the solution obtained from the step of replacing and then making up to final volume with buffer, wherein the concentration of the buffer is preferably about 10 mM to 20 mM, non-limiting examples of concentration of the buffer include 10 mM, 12 mM, 14 mM, 16 mM, 18 mM, and 20 mM; wherein the pH of the buffer is about 6.0 to 6.5, non-limiting examples of the pH of the buffer include 6.0, 6.1, 6.2, 6.3, 6.4, and 6.5.

The present invention further provides a method for preparing a lyophilized formulation comprising an anti-PCSK-9 antibody, which comprises the step of lyophilizing the pharmaceutical composition described above.

In an alternative embodiment, the method for preparing a lyophilized formulation containing an anti-PCSK-9 antibody sequentially comprises the steps of pre-freezing, primary drying, and secondary drying, wherein the pre-freezing is freezing from 5 °C to -40 °C- -50 °C, most preferably -45 °C, with no requirement for the condition of vacuum. The primary drying temperature is -14 ° C to 0 ° C, most preferably -5 ° C; the parameter of vacuum is 0.1 mBarto 0.5 mBar, most preferably 0.3 mBar. The secondary drying temperature is 20 °C to 30°C, preferably 25 °C, the degree of vacuum is 0.1 mBar to 0.5 mBar, most preferably 0.3 mBar, and the degree of vacuum is reduced to 0.005 mBar -0.02 mBar, and most preferably 0.01 mBar.

The present invention further provides a lyophilized formulation comprising an anti-PCSK-9 antibody prepared by the method for preparing a lyophilized formulation containing an anti-PCSK-9 antibody described above.

In some embodiments, the lyophilized formulation is stable at 2-8 ° C for at least 3 months, at least 6 months, at least 12 months, at least 18 months, or at least 24 months. In some embodiments, the lyophilized formulation is stable at 40 ° C for at least 7 days, at least 14 days or at least 28 days.

The present invention further provides a method for preparing a reconstituted solution of a lyophilized formulation comprising an anti-PCSK-9 antibody, comprising the step of reconstituting the lyophilized formulation described above, wherein the solvent for reconstitution is selected from, but not limited to water for injection, physiological saline or a solution of glucose.

The present invention further provides a reconstituted solution of a lyophilized formulation comprising an anti-PCSK-9 antibody prepared by the method for preparing a reconstituted solution of a lyophilized formulation comprising an anti-PCSK-9 antibody described above.

In an alternative embodiment, the reconstituted solution containing the anti-PCSK-9 antibody of the present invention comprises a component having a concentration of 2-5 times the concentration of the component contained in the pharmaceutical composition before lyophilization, preferably 3 times.

In an alternative embodiment, the reconstituted solution comprises the anti-PCSK-9 antibody of the present invention, wherein the concentration of the anti-PCSK9 antibody or the antigen binding fragment is about 120mg/ml to 200mg/ml, most preferably 150mg/ml.

In an alternative embodiment, for the reconstituted solution comprising the anti-PCSK-9 antibody of the present invention, the pH of the pharmaceutical composition is about 6.0-6.5, preferably 6.4. The pH of the reconstituted solution is related to the pH of the buffer used in the formulation of the drug substance. When the pH of the drug substance is 6.0, the pH of the final reconstituted solution is 6.3 ± 1.

In an alternative embodiment, for the reconstituted solution comprising the anti-PCSK-9 antibody of the present invention, the concentration of the buffer is about 15mM to 45mM, preferably 30mM.

In an alternative embodiment, the reconstituted solution comprising the anti-PCSK-9 antibody of the present invention further comprises disaccharide, wherein the disaccharide is preferably selected from the group consisting of trehalose and sucrose, most preferably sucrose.

In an alternative embodiment, for the reconstituted solution comprising the anti-PCSK-9 antibody of the present invention, the concentration of the disaccharide is about 55mg/ml to 95mg/ml, preferably about 75mg/ml.

In an alternative embodiment, the reconstituted solution comprising the anti-PCSK-9 antibody of the present invention further comprises a surfactant, wherein the surfactant is preferably polysorbate, more preferably polysorbate 80.

In an alternative embodiment, for the reconstituted solution comprising the anti-PCSK-9 antibody of the present invention, the concentration of the surfactant is about 0.4mg/ml to 0.8mg/ml, preferably 0.6mg/ml.

The invention further provides a product or kit, comprising a container containing any of the stable pharmaceutical compositions described herein. In some embodiments, it is a glass vial.

The invention further provides a use of the pharmaceutical composition, or the lyophilized formulation, or the reconstituted solution of the lyophilized formulation described above, in the formulation of a medicament for treating a PCSK9-mediated disease or disorder, wherein the disease or disorder is preferably a cholesterol-related disease; more preferably is selected from the group consisting of hypercholesterolemia, heart disease, metabolic syndrome, diabetes, coronary heart disease, apoplexy, cardiovascular disease, Alzheimer's disease and general Dyslipidemia; most preferably hypercholesterolemia, dyslipidemia, atherosclerosis, CVD or coronary heart disease.

The invention further provides a method for treating and preventing PCSK-9-related disease or disorder comprising administering to a patient in need thereof a therapeutically effective amount of the pharmaceutical composition or the lyophilized formulation or the reconstituted solution of the lyophilized formulation described above, wherein the disease or disorder is preferably a cholesterol-related disease; more preferably is selected from the group consisting of hypercholesterolemia, heart disease, metabolic syndrome, diabetes, coronary heart disease, apoplexy, cardiovascular disease, Alzheimer's disease and general Dyslipidemia; most preferably hypercholesterolemia, dyslipidemia, atherosclerosis, CVD or coronary heart disease.

The invention further provides a product, comprising a container containing the pharmaceutical composition or the lyophilized formulation or the reconstituted solution of the lyophilized formulation described above.

It should be understood that one, some, or all of the properties of the various embodiments described herein may be combined to form other embodiments of the present invention. These and other aspects of the invention will become apparent to a person skilled in the art. These and other embodiments of the invention are further described by the detailed description that follows.

### DETAILED DESCRIPTION OF THE INVENTION

In order that the present disclosure may be more readily understood, certain technical and scientific terms are specifically defined below. Unless specifically defined elsewhere in this document, all other technical and scientific terms used herein shall be taken to have the same meaning as commonly understood by one of ordinary skill in the art to which this invention belongs.

As used herein, "buffer" refers to a buffered solution that resists changes in pH by the action of its acid-base conjugate components. Examples of buffers that will control the pH in suitable range include acetate buffer, succinate buffer, gluconate buffer, histidine buffer, oxalate buffer, lactate buffer, phosphate buffer, citrate buffer, tartrate buffer, fumarate buffer, glycylglycine buffer and other organic acid buffers.

A "histidine buffer" is a buffer comprising histidine ions. Examples of the histidine buffers include histidine-hydrochloride buffer, histidine-acetate buffer, histidine-phosphate buffer and histidine-sulfate buffer, etc., preferably histidine-hydrochloride buffer. The histidine-hydrochloride buffer is prepared by histidine and hydrochloric acid, or histidine and histidine hydrochloride.

"Citrate buffer" is a buffer that includes citrate ions. Examples of the citrate buffer include citric acid-sodium citrate buffer, citric acid-potassium citrate buffer, citric acid-calcium citrate buffer and citric acid-magnesium citrate buffer, etc. A preferred citrate buffer is citric acid-sodium citrate buffer.

"Succinate buffer" is a buffer that includes succinate ions. Examples of the succinate buffer include succinic acid-sodium succinate buffer, succinic acid-potassium succinate buffer, succinic acid-calcium succinate buffer, etc. A preferred succinate buffer is succinic acid-succinate sodium buffer.

"Phosphate buffer" is a buffer that includes phosphate ions. Examples of the phosphate buffer solution include disodium hydrogen phosphate-sodium dihydrogen phosphate buffer and disodium hydrogen phosphate-potassium dihydrogen phosphate buffer, etc. A preferred phosphate buffer is disodium hydrogen phosphate-sodium dihydrogen phosphate buffer.

"Acetate buffer" is a buffer that includes acetate ions. Examples of the acetate buffer include acetic acid-sodium acetate buffer, acetate histidine buffer, acetic acid-potassium acetate buffer, acetic acid-calcium acetate buffer and acetic acid-magnesium acetate buffer, etc. A preferred acetate buffer is acetic acid-sodium acetate buffer.

"Pharmaceutical composition" refers to one containing a mixture of one or more compounds according to the present invention or a physiologically/pharmaceutically acceptable salt or produg thereof with other chemical components such as physiologically/pharmaceutically acceptable carriers and excipients. The pharmaceutical composition aims at maintaining the stability of the antibody active ingredient and promoting the administration to an organism, facilitating the absorption of the active ingredient and thereby exerting a biological effect. As used herein, "pharmaceutical composition" and "formulation" are not mutually exclusive.

"Lyophilized formulation" refers a formulation or pharmaceutical composition obtained by a vacuum freeze-drying step of a pharmaceutical composition in liquid or solution form or a liquid or solution formulation.

The lyophilization of the present invention includes pre-freezing, primary drying, and secondary drying. The purpose of pre-freezing is to freeze the product to obtain a crystalline solid. The pre-freezing temperature and the pre-freezing speed are two important process parameters. In the present invention, the pre-freezing temperature is set to -45 ° C, and the pre-freezing speed is set to 1 ° C / min. The primary drying is also known as sublimation, which is the main stage of lyophilization. The purpose of which is to maintain the shape of the product while removing the ice from the product, and minimizing damage to the product. If the temperature and vacuum degree of first drying are not appropriate, it will cause collapse of the product. Higher temperature and vacuum degree will accelerate the efficiency of lyophilization, but at the same time increase the risk of product collapsing. The temperature of the primary drying of the present invention may be a conventional temperature in the art, for example, -27 ° C to 0 ° C, preferably -14 ° C to -5 ° C. Secondary drying is also known as desorption, which is the main step to remove bound water from the product by pumping extreme vacuum (0.01 mbar) and increasing the temperature (20-40 °C). Since most biological products are sensitive to temperature, the temperature of secondary drying is set as the low point of the temperature range, i.e. 25 °C. The lyophilization time is related to freezer, dose of lyophilized formulation, and container of lyophilized formulation. Such timing adjustment of lyophilization is well known to those skilled in the art.

The pharmaceutical composition of the present invention is capable of achieving a stable effect: the antibody which can substantially retain its physical stability and/or chemical stability and/or biological activity after storage, preferably, the pharmaceutical composition substantially retains its physical stability, chemical stability and biological activity after storage. The storage time is generally selected based on the predetermined shelf life of the pharmaceutical composition. Currently, there are a number of analytical techniques for measuring protein stability that measure the stability after storage for a selected period of time at a selected temperature.

A "stable" pharmaceutical antibody formulation is a pharmaceutical antibody formulation without any observed significant changes at a refrigerated temperature (2-8°C) for at least 3 months, preferably 6 months, and more preferably 1 year, and even more preferably up to 2 years. Additionally, a "stable" liquid formulation includes one that exhibits desired features after storage for periods including 1 month, 3 months or 6 months at temperatures including at 25°C and 40°C. Typical acceptable criteria for stability are as follows: typically, no more than about 10%, preferably about 5%, of the antibody monomer is degraded as measured by SEC-HPLC. The pharmaceutical antibody formulation is colorless, or clear to slightly opalescent by visual analysis. The concentration, pH and osmolality of the formulation have no more than +/-10% change. Typically, no more than about 10%, preferably about 5% of clipping is observed. Typically, no more than about 10%, preferably about 5% of aggregation is formed.

An antibody "retains its physical stability" in a pharmaceutical formulation if it shows no significant increase of aggregation, precipitation and/or denaturation upon visual examination of color and/or clarity, or as measured by UV light scattering, size exclusion chromatography (SEC) and dynamic light scattering (DLS). The changes of protein conformation can be evaluated by fluorescence spectroscopy, which determines the protein tertiary structure, and by FTIR spectroscopy, which determines the protein secondary structure.

An antibody "retains its chemical stability" in a pharmaceutical formulation, if it shows no significant chemical alteration. Chemical stability can be assessed by detecting and quantifying chemically altered forms of the protein. Degradation processes that often alter the protein chemical structure include hydrolysis or clipping (evaluated by methods such as size exclusion chromatography and SDS-PAGE, etc.), oxidation (evaluated by methods such as peptide mapping in conjunction with mass spectroscopy or MALDI/TOF/MS, etc.), deamidation (evaluated by methods such as ion-exchange chromatography, capillary isoelectric focusing, peptide mapping, isoaspartic acid measurement, etc.), and isomerization (evaluated by measuring the content of isoaspartic acid, peptide mapping, etc.).

An antibody "retains its biological activity" in a pharmaceutical formulation, if the biological activity of the antibody at a given time is within a predetermined range of the biological activity exhibited at the time the pharmaceutical formulation was prepared. The biological activity of an antibody can be determined, for example, by an antigen binding assay.

As used herein, the single-letter code and the three-letter code for amino acids are as described in J. Biol. Chem, 243, (1968) p3558*.*

As used herein, "Antibody" refers to immunoglobulin, a four-peptide chain structure connected together by disulfide bonds between two identical heavy chains and two identical light chains.

In the present invention, the antibody light chain mentioned herein further comprises a light chain constant region, which comprises a human or murine κ, λ chain or a variant thereof.

In the present invention, the antibody heavy chain mentioned herein further comprises a heavy chain constant region, which comprises human or murine IgG1, 2, 3, 4 or a variant thereof.

The variable region (Fv region), comprises about 110 of amino acids close to the N-terminus of the antibody heavy and light chain. The constant region (C region), comprising the remaining amino acids close to the C-terminus of the antibody, is relatively stable. The variable region comprises three hypervariable regions (HVRs) and four relatively conserved framework regions (FRs). The three hypervariable regions, which determine the specificity of the antibody, are also termed complementarity determining regions (CDRs). Each of the light chain variable region (LCVR) and the heavy chain variable region (HCVR) is composed of three CDR regions and four FR regions, arranged from amino terminus to carboxy terminus in the following order: FR1, CDR1, FR2, CDR2, FR3, CDR3, and FR4. Three light chain CDRs refer to LCDR1, LCDR2, and LCDR3; three heavy chain CDRs refer to HCDR1, HCDR2 and HCDR3. The number and location of CDR amino acid residues in LCVR and HCVR regions of the antibody or the antigen binding fragments herein comply with known Kabat numbering criteria (LCDR1-3, HCDE2-3), or comply with Kabat and Chothia numbering criteria (HCDR1).

The antibody of the present invention comprises a murine antibody, a chimeric antibody or a humanized antibody, preferably a humanized antibody.

The term "murine antibody" in the present invention refers to an anti-human PCSK9 monoclonal antibody prepared according to the knowledge and skill in the art. During the formulation, a test object is injected with a PCSK9 antigen, and then a hybridoma expressing antibody which possesses desired sequences or functional characteristics is isolated.

The term "chimeric antibody", is an antibody formed by fusing a variable region of a murine antibody with the constant region of a human antibody, which can alleviate immune response induced by the murine antibody. To construct the chimeric antibody, a hybridoma that secretes a murine-specific monoclonal antibody is first established, then a variable region gene is cloned from the murine hybridoma cells. Subsequently, a constant region gene of the human antibody is cloned as desired. The murine variable region gene is ligated with human constant region gene to form a chimeric gene which is then inserted into a human vector, and finally the chimeric antibody molecule is expressed in the eukaryotic or prokaryotic industrial system. In a preferred embodiment of the present invention, the light chain of the anti-PCSK9 chimeric antibody further comprises the light chain constant regions of human κ, λ chain or a variant thereof. The heavy chain of the anti-PCSK9 chimeric antibody further comprises the heavy chain constant regions of human IgG1, IgG2, IgG3 or IgG4, or variants thereof. The constant region of a human antibody may be selected from the heavy chain constant region of human IgGl, IgG2, IgG3 or IgG4 or variants thereof, preferably comprising the heavy chain constant region of human IgG2 or IgG4, or IgG4 without ADCC (antibody-dependent cell-mediated cytotoxicity) toxicity after amino acid mutation.

The term "humanized antibody", also known as CDR-grafted antibody, refers to an antibody generated by grafting murine CDR sequences into a variable region framework of a human antibody, namely, an antibody produced from different types of human antibody framework sequences. A humanized antibody overcomes the disadvantage of the strong antibody response induced by the chimeric antibody which carries a large amount of murine protein components. Such framework sequences can be obtained from public DNA database covering germline antibody gene sequences or published references. For example, germline DNA sequences of human heavy and light chain variable region genes can be found in "VBase" human germline sequence database (available on web www.mrccpe.com.ac.uk/vbase), as well as Kabat, EA, et al, 1991 Sequences of Proteins of Immunological Interest, 5th Ed*.* To avoid the decrease in activity along with the decrease in immunogenicity, the framework sequences in the variable region of the human antibody are subjected to minimal back mutations to maintain the activity. The humanized antibody of the present invention also comprises a humanized antibody to which CDR affinity maturation is performed by phage display.

An "anti-PCSK-9 antibody" is an antibody that specifically binds to PCSK-9, including but not limited to the h001 series of PCSK-9 humanized antibodies of PCT/CN2016/111053. Wherein, the h001 series of PCSK-9 humanized antibodies was screened by human PCSK-9 immunized mice and obtained by humanized transformation.

"Antigen-binding fragment" in the present invention refers to Fab fragment, Fab' fragment, or F(ab')2 fragment having antigen-binding activity, as well as scFv fragment binding to human PCSK9 and other fragments capable of binding PCSK9, which are formed by VH and VL regions of PCSK9 binding antibodies; it comprises one or more CDR regions selected from the group consisting of SEQ ID NO: 12 to SEQ ID NO: 17 of antibodies described in the present invention. Without a constant region, an Fv fragment comprises heavy chain variable region and light chain variable region, which is a minimal antibody fragment possessing all antigen-binding sites. Generally, an Fv antibody further comprises a polypeptide linker between the VH and VL domains, and is capable of forming a structure necessary for antigen binding. Also, different linkers can be used to connect the variable regions of two antibodies to form a polypeptide chain, named single chain antibody or single chain Fv (scFv). The term "binding to PCSK-9" in this invention means that it's capable of interacting with human PCSK-9. The term "antigen-binding sites" in the present invention, refers to continuous or discontinuous, three-dimensional sites on the antigen, recognized by the antibody or the antigen-binding fragment of the present invention.

Methods for producing and purifying antibodies and antigen-binding fragments are well known in the art and can be found, for example, in Antibody Experimental Technology Guide of Cold Spring Harbor, Chapters 5-8 and 15. The antibody or the antigen-binding fragments of the present invention is genetically engineered to introduce one or more human framework regions (FRs) to a non-human derived CDR. Human FR germline sequences can be obtained by comparing ImMunoGeneTics (IMGT) human antibody variable region germline gene database with MOE software from IMGT via their website, or from The Immunoglobulin FactsBook, 2001ISBN012441351.

The engineered antibody or antigen-binding fragments of the present invention may be prepared and purified using conventional methods. For example, cDNA sequences encoding a heavy chain and a light chain may be cloned and recombined into a GS expression vector. A recombined immunoglobulin expression vector may be stably transfected into a CHO cell. As a more recommended method well known in the art, mammalian expression systems will result in glycosylation of antibodies, typically at the highly conserved N-terminus in the Fc region. Stable clones may be obtained through expression of an antibody specifically binding to human PCSK-9. Positive clones may be expanded in serum-free culture medium for antibody production in bioreactors. Culture medium, into which an antibody has been secreted, may be purified by conventional techniques. For example, the medium may be conveniently applied to a Protein A or G Sepharose FF column that has been equilibrated with adjusted buffer. The column is washed to remove nonspecific binding components. The bound antibody is eluted by pH gradient and then pooled, and the antibody fragments are determined by SDS-PAGE. The antibody may be filtered and concentrated using conventional techniques. Soluble aggregate and multimers may be effectively removed by conventional techniques, including size exclusion or ion exchange. The obtained product may be immediately frozen, for example at -70°C, or may be lyophilized.

"Conservative modifications" or "conservative replacement or substitution" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. It is known by those skilled in this art that, in general, single amino acid substitution in non-essential regions of a polypeptide does not substantially alter biological activity of the polypeptide (see, e.g., Watson et al. (1987) Molecular Biology of the Gene, The Benjamin/Cummings Pub. Co., p.224 (4.sup.th Ed*.)).* In addition, substitutions of structurally or functionally similar amino acids are less likely to disrupt biological activity.

Amino acid "identity" refers to sequence similarity between two protein sequences or between two polypeptide sequences. When a position in both of the two compared sequences is occupied by the same amino acid residue, then the molecules are the same at that position. Examples of algorithms suitable for determining percent sequence identity and percent sequence similarity are the BLAST and BLAST 2.0 algorithms, which were described in Altschul et al. (1990) J. Mol. Biol. 215: 403-410 and Altschul et al, respectively. (1977) Nucleic Acids Res. 25:3389-3402*.* Software for performing BLAST analyses is available at the National Center for Biotechnology Information (www.ncbi.nlm.nih.gov/).

"Administration" and "treatment," when applying to an animal, human, experimental subject, cell, tissue, organ, or biological fluid, refer to contacting an exogenous pharmaceutical, therapeutic, diagnostic agent, or composition with the animal, human, subject, cell, tissue, organ, or biological fluid. "Administration" and "treatment" can refer to, e.g., therapeutic, pharmacokinetic, diagnostic, research, and experimental methods. Treatment of a cell encompasses contacting a reagent with the cell, as well as contacting a reagent with a fluid, where the fluid is in contact with the cells. "Administration" and "treatment" also mean *in vitro* and *ex vivo* treatments, e.g., of a cell, by a reagent, diagnostic, binding compound, or by another cell. "Treatment," as it applies to a human, veterinary, or a research subject, refers to therapeutic treatment, prophylactic or preventative measures, or research and diagnostic applications.

"Therapy" means to administer a therapeutic agent, such as a composition comprising any of the binding compounds of the present invention, internally or externally to a patient having one or more disease symptoms for which the agent has known therapeutic activity. Typically, the therapeutic agent is administered in an amount effective to alleviate one or more disease symptoms in a subject or population to be treated, so as to induce the regression of or inhibit the progression of such symptom(s) to any clinically measurable degree. The amount of a therapeutic agent that is effective to alleviate any particular disease symptom (also referred to "therapeutically effective amount") may vary depending on a variety of factors, such as disease state, age, weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. Although embodiments of the present invention (e.g., therapeutic methods or articles of manufacture) may not be effective in alleviating the disease symptom(s) of interest in every patient, it should alleviate the target disease symptom(s) of interest in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Effective amount" encompasses an amount sufficient to ameliorate or prevent a symptom or sign of a medical condition. Effective amount also means an amount sufficient to allow or facilitate diagnosis. An effective amount for a particular patient or veterinary subject may vary depending on factors such as the condition being treated, the general health of the patient, the route and dose of administration and the severity of side effects. An effective amount can be the maximal dose or dosing protocol that avoids significant side effects or toxic effects.

The "Tm value" refers to the heat denaturation temperature of the protein, that is, the temperature at which half of the protein is unfolded, and the spatial structure of the protein is destroyed at this time, therefore the higher the Tm value, the higher the thermal stability of the protein.

"Replacement" refers to the replacement of a solvent system that dissolves antibody proteins. For example, a buffer system of stable formulation is used to replace the high salt or hypertonic solvent system containing the antibody protein by physical modes of operation. Wherein the physical modes of operation include, but are not limited to, ultrafiltration, dialysis, or reconstitution after centrifugation.

### EXAMPLES AND TESTS

Hereinafter, the present invention is further described in detail with reference to the following examples, however, these examples are for illustrative purposes only and are not intended to limit the scope of the present invention.

In the examples of the present invention, where specific conditions are not described, the experiments are generally conducted under conventional conditions, or under conditions proposed by the material or product manufacturers. Where the source of the reagents is not specifically given, the reagents are commercially available conventional reagents.

### EXAMPLES

### Formulation of PCSK-9 antigen and antibody

### Example 1. Formulation of PCSK9 Antigen and Test Protein

### Protein design and expression

Uniprot Proprotein convertase subtilisin/kexin type 9 (human PCSK9, Uniprot number: Q8MBP7) was used as a template for PCSK9 of the invention to design the amino acid sequences of antigen and test protein. PCSK9 proteins were fused with different labels such as a His tag or an immune-promoting peptide such as PADRE peptide, then cloned into pTT5 vectors (Biovector, Cat#: 102762) or pTargeT vectors (promega, A1410), respectively. The PCSK9 proteins were then transiently expressed in 293 cells or stably expressed in CHO-S cells and purified. Finally, the antigen and test protein of the invention were obtained.

PCSK9 with His tag: PCSK9-His6, used as an immunogen for immunizing mice or a detection reagent, is as follows: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAAR
RGYLTKILHVFHGLLPGFLVKMSGDLLELALKLPHVDYIEEDSSVFAQSIPWNL
ERITPPRYRADEYQPPDGGSLVEVYLLDTSIQSDHREIEGRVMVTDFENVPEED
GTRFHRQASKCDSHGTHLAGVVSGRDAGVAKGASMRSLRVLNCQGKGTVSG
TLIGLEFIRKSQLVQPVGPLVVLLPLAGGYSRVLNAACQRLARAGVVLVTAAG
NFRDDACLYSPASAPEVITVGATNAQDQPVTLGTLGTNFGRCVDLFAPGEDIIG
ASSDCSTCFVSQSGTSQAAAHVAGIAAMMLSAEPELTLAELRQRLIHFSAKDV
INEAWFPEDQRVLTPNLVAALPPSTHGAGWQLFCRTVWSAHSGPTRMATAVAR
CAPDEELLSCSSFSRSGKRRGERMEAQGGKLVCRAHNAFGGEGVYAIARCCL
LPQANCSVHTAPPAEASMGTRVHCHQQGHVLTGCSSHWEVEDLGTHKPPVLR
PRGQPNQCVGHREASIHASCCHAPGLECKVKEHGIPAPQEQVTVACEEGWTL
TGCSALPGTSHVLGAYAVDNTCVVRSRDVSTTGSTSEGAVTAVAICCRSRHLA
QASQELQHHHHHH

SEQ ID NO: 1
 Note: Underlined sequence is a signal peptide, and the italic part is His-tag sequence (His6- tag).

PCSK9 with PADRE peptide and His-tag: namely PCSK9-PADRE-His6, which is used as an immunogen, containing PADRE peptide that can promote immunization: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAAR
RGYLTKILHVFHGLLPGFLVKMSGDLLELALKLPHVDYIEEDSSVFAQSIPWNL
ERITPPRYRADEYQPPDGGSLVEVYLLDTSIQSDHREIEGRVMVTDFENVPEED
GTRFHRQASKCDSHGTHLAGVVSGRDAGVAKGASMRSLRVLNCQGKGTVSG
 
TLIGLEFIRKSQLVQPVGPLVVLLPLAGGYSRVLNAACQRLARAGVVLVTAAG
NFRDDACLYSPASAPEVITVGATNAQDQPVTLGTLGTNFGRCVDLFAPGEDIIG
ASSDCSTCFVSQSGTSQAAAHVAGIAAMMLSAEPELTLAELRQRLIHFSAKDV
INEAWFPEDQRVLTPNLVAALPPSTHGAGWQLFCRTVWSAHSGPTRMATAVAR
CAPDEELLSCSSFSRSGKRRGERMEAQGGKLVCRAHNAFGGEGVYAIARCCL
LPQANCSVHTAPPAEASMGTRVHCHQQGHVLTGCSSHWEVEDLGTHKPPVLR
PRGQPNQCVGHREASIHASCCHAPGLECKVKEHGIPAPQEQVTVACEEGWTL
TGCSALPGTSHVLGAYAVDNTCVVRSRDVSTTGSTSEGAVTAVAICCRSRHLA
QASQELQGSGAKFVAAWTLKAAAHHHHHH
SEQ ID NO: 2
 Note: The underlined sequence is a signal peptide, the double underlined sequence is a linker, the dashed line sequence is PADRE peptide, and the italic part is His6-tag.

A fusion protein of His tag and PCSK9 with TEV cleavage site: PCSK9-TEV-His6, N-PCSK9 (N terminal PCSK9 domain), can be obtained by TEV cleavage and used as an immunogen: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAAR
RGYLTKILHVFHGLLPGFLVKMSGDLLELALKLPHVDYIEEDSSVFAQSIPWNL
ERITPPRYRADEYQPPDGGSLVEVYLLDTSIQSDHREIEGRVMVTDFENVPEED
GTRFHRQASKCDSHGTHLAGVVSGRDAGVAKGASMRSLRVLNCQGKGTVSG
TLIGLEFIRKSQLVQPVGPLVVLLPLAGGYSRVLNAACQRLARAGVVLVTAAG
NFRDDACLYSPASAPEVITVGATNAQDQPVTLGTLGTNFGRCVDLFAPGEDIIG
ASSDCSTCFVSQSGTSQAAAHVAGIAAMMLSAEPELTLAELRQRLIHFSAKDV
INEAWFPEDQRVLTPNLVAALPPSTHENLYFQGAGWQLFCRTVWSAHSGPTR
MATAVARCAPDEELLSCSSFSRSGKRRGERMEAQGGKLVCRAHNAFGGEGVY
AIARCCLLPQANCSVHTAPPAEASMGTRVHCHQQGHVLTGCSSHWEVEDLGT
HKPPVLRPRGQPNQCVGHREASIHASCCHAPGLECKVKEHGIPAPQEQVTVAC
EEGWTLTGCSALPGTSHVLGAYAVDNTCVVRSRDVSTTGSTSEGAVTAVAICC
 
RSRHLAQASQELQHHHHHH
SEQ ID NO: 3
 Note: The underlined sequence is a signal peptide, the double underlined sequence is TEV cleavage site, and the italic part is His6-tag.

PCSK9-D374Y mutant protein, with His-tag: PCSK9-D374Y-His6, which is used as a test reagent: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAAR
RGYLTKILHVFHGLLPGFLVKMSGDLLELALKLPHVDYIEEDSSVFAQSIPWNL
ERITPPRYRADEYQPPDGGSLVEVYLLDTSIQSDHREIEGRVMVTDFENVPEED
GTRFHRQASKCDSHGTHLAGVVSGRDAGVAKGASMRSLRVLNCQGKGTVSG
TLIGLEFIRKSQLVQPVGPLVVLLPLAGGYSRVLNAACQRLARAGVVLVTAAG
NFRDDACLYSPASAPEVITVGATNAQDQPVTLGTLGTNFGRCVDLFAPGEDIIG
ASSYCSTCFVSQSGTSQAAAHVAGIAAMMLSAEPELTLAELRQRLIHFSAKDV
INEAWFPEDQRVLTPNLVAALPPSTHGAGWQLFCRTVWSAHSGPTRMATAVAR
CAPDEELLSCSSFSRSGKRRGERMEAQGGKLVCRAHNAFGGEGVYAIARCCL
LPQANCSVHTAPPAEASMGTRVHCHQQGHVLTGCSSHWEVEDLGTHKPPVLR
PRGQPNQCVGHREASIHASCCHAPGLECKVKEHGIPAPQEQVTVACEEGWTL
TGCSALPGTSHVLGAYAVDNTCVVRSRDVSTTGSTSEGAVTAVAICCRSRHLA
QASQELQHHHHHH

SEQ ID NO: 4
 Note: The underlined sequence is a signal peptide, and the italic part is His6-tag.

PCSK9 protein inserted with biotin receiving peptide BP15 and His tag, namely PCSK9-BP15-His6. As a test reagent, BP15 peptide position can be biotinylated during expression, thus avoiding the biotin labeling *in vitro* and possible conformational changes. MGTVSSRRSWWPLPLLLLLLLLLGPAGARAQEDEDGDYEELVLALRSEEDGL 
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVL
VTAAGNFRDDACLYSPASAPEVITVGATNAQDQPVTLGTLGTNFGRCVDLFA
PGEDIIGASSDCSTCFVSQSGTSQAAAHVAGIAAMMLSAEPELTLAELRQRLIH
FSAKDVINEAWFPEDQRVLTPNLVAALPPSTHGAGWQLFCRTVWSAHSGPTR
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQGSTSGSGLNDIFEAQKIEWHEHHHHHH
SEQ ID NO: 5
 NOTE: The underlined sequence is a signal peptide, the double underlined sequence is the biotin receiving peptide, and the italic part is His6-tag.

PCSK9-Y is the abbreviation of PCSK9 D374Y mutant protein inserted with biotin receiving peptide BP15 and His tag, namely PCSK9-D374Y-BP15-His6, which is used as a test protein: 
MGTVSSRRSWWPLPLLLLLLLLLGPAGARAQEDEDGDYEELVLALRSEEDGL
AEAPEHGTTATFHRCAKDPWRLPGTYVVVLKEETHLSQSERTARRLQAQAA
RRGYLTKILHVFHGLLPGFLVKMSGDLLELALKLPHVDYIEEDSSVFAQSIPW
NLERITPPRYRADEYQPPDGGSLVEVYLLDTSIQSDHREIEGRVMVTDFENVP
EEDGTRFHRQASKCDSHGTHLAGVVSGRDAGVAKGASMRSLRVLNCQGKG
TVSGTLIGLEFIRKSQLVQPVGPLVVLLPLAGGYSRVLNAACQRLARAGVVL
VTAAGNFRDDACLYSPASAPEVITVGATNAQDQPVTLGTLGTNFGRCVDLFA
PGEDIIGASSYCSTCFVSQSGTSQAAAHVAGIAAMMLSAEPELTLAELRQRLIH
FSAKDVINEAWFPEDQRVLTPNLVAALPPSTHGAGWQLFCRTVWSAHSGPTR
 
MATAVARCAPDEELLSCSSFSRSGKRRGERMEAQGGKLVCRAHNAFGGEGV
YAIARCCLLPQANCSVHTAPPAEASMGTRVHCHQQGHVLTGCSSHWEVEDL
GTHKPPVLRPRGQPNQCVGHREASIHASCCHAPGLECKVKEHGIPAPQEQVT
VACEEGWTLTGCSALPGTSHVLGAYAVDNTCVVRSRDVSTTGSTSEGAVTA
VAICCRSRHLAQASQELQGSTSGSGLNDIFEAQKIEWHEHHHHHH
SEQ ID NO: 6
 NOTE: The underlined sequence is a signal peptide, the double underlined sequence is biotin receiving peptide, and the italic part is His6-tag.

LDLR extracellular domain of PCSK9 receptor protein with Flag tag and His tag, namely LDLR-ECD-Flag-His6, used as a test reagent: 
MGPWGWKLRWTVALLLAAAGTAVGDRCERNEFQCQDGKCISYKWVCDGS
AECQDGSDESQETCLSVTCKSGDFSCGGRVNRCIPQFWRCDGQVDCDNGSDE
QGCPPKTCSQDEFRCHDGKCISRQFVCDSDRDCLDGSDEASCPVLTCGPASFQ
CNSSTCIPQLWACDNDPDCEDGSDEWPQRCRGLYVFQGDSSPCSAFEFHCLS
GECIHSSWRCDGGPDCKDKSDEENCAVATCRPDEFQCSDGNCIHGSRQCDRE
YDCKDMSDEVGCVNVTLCEGPNKFKCHSGECITLDKVCNMARDCRDWSDE
PIKECGTNECLDNNGGCSHVCNDLKIGYECLCPDGFQLVAQRRCEDIDECQD
PDTCSQLCVNLEGGYKCQCEEGFQLDPHTKACKAVGSIAYLFFTNRHEVRKM
TLDRSEYTSLIPNLRNVVALDTEVASNRIYWSDLSQRMICSTQLDRAHGVSSY
DTVISRDIQAPDGLAVDWIHSNIYWTDSVLGTVSVADTKGVKRKTLFRENGS
KPRAIVVDPVHGFMYWTDWGTPAKIKKGGLNGVDIYSLVTENIQWPNGITLD
LLSGRLYWVDSKLHSISSIDVNGGNRKTILEDEKRLAHPFSLAVFEDKVFWTD
IINEAIFSANRLTGSDVNLLAENLLSPEDMVLFHNLTQPRGVNWCERTTLSNG
GCQYLCLPAPQINPHSPKFTCACPDGMLLARDMRSCLTEAEAAVATQETSTV
RLKVSSTAVRTQHTTTRPVPDTSRLPGATPGLTTVEIVTMSHQALGDVAGRG
NEKKPSSVRDYKDDDDKHHHHHH
SEQ ID NO: 7
 NOTE: The underlined sequence is a signal peptide, the double underlined sequence is Flag tag, and the italic part is His6-tag.

LCDR-Fc, a fusion protein of truncated LDLR extracellular domain with hIgG1 Fc (with PCSK9 binding activity): namely LDLR-sECD -Fc (hIgG1), used as a test reagent: 
MEFGLSWLFLVAILKGVQCGTNECLDNNGGCSHVCNDLKIGYECLCPDGFQL
VAQRRCEDIDECQDPDTCSQLCVNLEGGYKCQCEEGFQLDPHTKACKEPKSS
DKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSL
SPGK
SEQ ID NO: 8
 NOTE: The underlined sequence is a signal peptide, the double underlined sequence is truncated LDLR extracellular domain with PCSK9 binding activity (LDLR-sECD), and the italic part is hIgG1-Fc.

A fusion protein of a more truncated LDLR extracellular domain with a hIgG1 Fc (with PCSK9 binding activity): namely LDLR-ssECD -Fc (hIgG1), used as a detection reagent: 
MEFGLSWLFLVAILKGVQCGTNECLDNNGGCSHVCNDLKIGYECLCPDGFQL
VAQRRCEDIDEPKSSDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSV
MHEALHNHYTQKSLSLSPGK
SEQ ID NO: 9
 NOTE: The underlined sequence is a signal peptide, the double underlined sequence is the more truncated LDLR extracellular domain with PCSK9 binding activity (LDLR-ssECD), and the italic part is the hIgG1-Fc.

### Example 2. Purified Recombinant Protein of PCSK9 and LDLR Related Recombinant Protein, and Purification of Hybridoma Antibody and Recombinant Antibody

### 1. Purification steps of recombinant proteins with His-tag:

The samples of cell expression supernatant were centrifuged at high-speed centrifugation and impurities were removed. The buffer was replaced with PBS, and imidazole was added to a final concentration of 5 mM. A nickel column was equilibrated with 2-5 column volumes of PBS solution containing 5 mM imidazole. After buffer replacement, the supernatant sample was loaded onto the immobilized metal affinity chromatography (IMAC) column. The column was washed with PBS solution containing 5 mM imidazole, until the readout at A₂₈₀ was reduced to baseline. Then, the chromatographic column was washed with PBS + 10 mM imidazole to remove nonspecific binding proteins and efflux was collected. The target protein was eluted with PBS solution containing 300 mM imidazole and the elution peak was collected. The collected elution was concentrated and further purified by gel chromatography Superdex 200 (GE) and the mobile phase was PBS. The multimer peak was removed and the elution peaks were collected. The obtained proteins were identified by electrophoresis, peptide mapping and LC-MS. PCSK9-His6 (SEQ ID NO:1), PCSK9-PADRE-His6 (SEQ ID NO: 2), PCSK9-TEV-His6 (SEQ ID NO: 3), PCSK9-D374Y-His6 (SEQ ID NO: 4), PCSK9-BP15-His6 (SEQ ID NO: 5), and PCSK9-D374Y-BP15-His6 (SEQ ID NO: 6) were obtained and used as the immunogen or test protein of the invention. PCSK9-TEV-His6 was purified and cleaved by TEV enzyme. The TEV enzyme, incompletely cleaved PCSK9-TEV-His6, or C-terminal domain fragment with His-tag was removed from the obtained product via IMAC column. The IMAC effluent was concentrated to obtain a PCSK9 segment with N-terminus domain only (abbreviated as N-PCSK9), and used as an immunogen for immunizing mice.

### 2. Purification steps of recombinant protein of LDLR-ECD-Flag-His6 (SEQ ID NO: 7) with His tag and Flag tag:

Samples were centrifuged at high-speed to remove impurities, and then concentrated to a proper volume. Flag Affinity Column was equilibrated with 2-5 column volumes of 0.5×PBS buffer. After the impurities was removed, the samples of cell expression supernatant were loaded onto the column. The column was washed with 0.5×PBS, until the readout at A₂₈₀ was reduced to the baseline. The column was washed with PBS containing 0.3 M NaCl, and impurity proteins were eluted and collected. Target proteins were eluted with 0.1 M acetic acid (pH 3.5-4.0) and collected, and then pH value of which was adjusted to neutral. The collected elution was concentrated and further purified by gel chromatography Superdex 200 (GE) and the mobile phase was PBS. The multimer peak was removed and the elution peaks were collected. The obtained proteins were identified by electrophoresis, peptide mapping and LC-MS, and aliquoted for later use. LDLR-ECD-Flag-His6 (SEQ ID NO: 7) with FLAG/His6 tags were obtained and used for performance testing of the antibody of the present invention.

### 3. Purification steps of fusion protein of LDLR Fc:

Samples of cell expression supernatant were centrifuged at high-speed to remove impurities, and then samples were concentrated to a proper volume and loaded onto Protein A column. The column was washed with PBS until the readout at A₂₈₀ was reduced to the baseline. Target proteins were eluted with 100 mM sodium acetate, pH 3.0 and then neutralized with 1 M Tris-HCl. The eluted samples were properly concentrated and were further purified by gel chromatography Superdex 200 (GE) pre-equilibrated with PBS. The peaks without multimer were collected. This method was used to purify LDLR-sECD -Fc (hIgG1) (SEQ ID NO: 8) and LDLR-ssECD -Fc (hIgG1) (SEQ ID NO: 9), both of which can be used for performance testing of anti-PCSK9 antibodies.

### Example 3. Preparation of Anti-human PCSK9 Hybridoma Monoclonal antibodies

### 1. Immunization

The anti-human PCSK9 monoclonal antibody was produced by immunizing mice. SJL white mice, female, 6 weeks old (Beijing Vital River Laboratory Animal Technology Co., Ltd., animal permit number: SCXK (Beijing) 2012-0001) were used in this experiment and raised in a SPF laboratory. After purchase, the mice were kept in the laboratory for 1 week under 12/12 hours light/dark cycle, at a temperature of 20-25°C, and humidity 40-60%. The mice that had been adapted to the environment were immunized according to the following two schemes, with 6-10 mice per group. Immunogens were human PCSK9-His6 (SEQ ID NO: 1) with His tag, PCSK9-PADRE-His6 (SEQ ID NO: 2) and N-PCSK9 (SEQ ID NO: 3).
Scheme A: emulsifying with Freund's adjuvant (sigma Lot Num: F5881/F5506): Complete Freund's adjuvant (CFA) was used for primary immunization and Incomplete Freund's adjuvant (IFA) was used for boost immunization. The ratio of antigen to adjuvant was 1:1, 100 µg/mouse for the first immunization, and 50 µg/mouse for the booster immunization. On day 0, each mouse was intraperitoneally (IP) injected with 100 µg of emulsified antigens, once every two weeks after primary immunization, for a total of 6-8 weeks.
Scheme B: Mice were cross immunized with Titermax (sigma Lot Num: T2684) and Alum (Thremo Lot Num: 77161). The ratio of antigen to adjuvant (titermax) was 1:1, and the ratio of antigen to adjuvant (Alum) was 3:1, 10-20µg/mouse for first immunization, and 5 µg/mouse for booster immunization. On day 0, each mouse was intraperitoneally (IP) injected with 20/10 µg emulsified antigens, once a week after primary immunization, Titermax and Alum were used alternately for a total of 6-11 weeks. Four weeks after immunization, the antigen was back or intraperitoneally injected according to the swelling conditions on the back and abdomen.

### 2. Cell fusion

Mice with high antibody titer in serum (See Tests 1 and 2, ELISA method for binding PCSK9) and with the titer tending to be stationary were chosen for splenocyte fusion. 72 hours prior to fusion, the chosen mice were immunized with PCSK9-His6 via intraperitoneal injection, 10 µg/mouse. The spleen lymphocytes and myeloma cells Sp2/0 (ATCC® CRL-8287™) were fused to obtain hybridoma cells by optimized PEG-mediated fusion procedure. The fused hybridoma cells were re-suspended with HAT complete medium (RPMI-1640 medium containing 20%FBS, 1×HAT and 1×OPI), and then aliquoted into a 96-well cell culture plate (1×10⁵/150µl/well) and incubated at 37°C and 5% CO₂. On day 5 after fusion, HAT complete medium was added with 50µlwell, and incubated at 37°C and 5%CO₂. On day 7 to day 8 after fusion, based on cells growth density, the whole medium was exchanged to HT complete medium (RPMI-1640 medium containing 20%FBS, 1×HT and 1×OPI), 200µ/well, and incubated at 37°C and 5% CO₂.

### 3. Screening of hybridoma cells

On day 10 to day 11 after fusion, based on cell growth density, ELISA tests for PCSK9 or PCSK9-Y binding were performed (See Tests 1 and 2). Positive cells tested with ELISA were detected by blocking ELISA of PCSK9 or PCSK9-Y binding to LDLR (See Tests 3 and 4). The medium in the positive wells was exchanged and the cells were expanded to 24-well plates promptly based on cell density. Upon retest, the cell strains transferred into 24-well plate were preserved and subjected to first sub-clone. The positive cells after the first sub-clone screening (See Tests 1 and 2) were preserved and subjected to the second sub-clone. The positive cells after the second sub-clone (See Tests 1 and 2) were preserved and subjected to protein expression. Upon multiple fusions, hybridoma cells capable of blocking the binding of PCSK9 or PCSK9-Y to LDLR were obtained.

The hybridoma clone mAb-001 was obtained by screening according to blocking assay and binding assay. The antibody was further prepared by serum-free cell culturing, and the antibody was purified according to purification example for use in the test example.

The murine antibody variable region sequence of the hybridoma clone mAb-001 was as follows:
> mAb-001 VH 
QVHLQQSGAELAKPGASVKLSCKASGYTFNDYWMHWVKERPGQGLEWIGY
INPSSGFTKYHQNFKDKATLTADKSSSTAYMQLSSLTYDDSAVYYCARQYDYDED
WYFDVWGTGTTVTVSS
SEQ ID NO: 10
> mAb-001VL 
DIVMSQSPSSLAVSAGEKVTMSCKSSQSLLNSRTRKNFLAWYQQKPGQSPKL
LIYWASTRESGVPDRFTGRGSGTDFTLTISSVQAEDLAVYYCKQSFNLFTFGSGTK
LEIK
SEQ ID NO: 11
Note: The order is FR1-CDR1-FR2-CDR2-FR3-CDR3-FR4, the italic part is FR sequence, and the underlined is CDR sequence.

**Table 1. CDR region sequences of heavy chain and light chain in Anti-PCSK-9 antibody mAb-001**

| | Heavy Chain | | Light Chain | |
|---|---|---|---|---|
| mAb -001 | HCDR1 | DYWMH SEQ ID NO: 12 | LCDR1 | KSSQSLLNSRTRKNFLA SEQ ID NO: 15 |
| | HCDR2 | YINPSSGFTKYHQNFKD SEQ ID NO: 13 | LCDR2 | WASTRES SEQ ID NO: 16 |
| | HCDR3 | QYDYDEDWYFDV SEQ ID NO: 14 | LCDR3 | KQSFNLFT SEQ ID NO: 17 |

### Example 4. Humanization of Anti-human PCSK9 Hybridoma Monoclonal Antibody

1. Selection of humanized framework for hybridoma clone mAb-001
By aligning IMGT human antibody heavy and light chain variable region germline gene database and MOE software, the heavy and light chain variable region genes with high homology with mAb-001 were selected as templates respectively. The CDRs of these two murine antibodies were respectively grafted into the corresponding human templates to form variable region sequences with the order of FR1-CDR1-FR2-CDR2-FR3-CDR3-FR4. Wherein, the amino acid residues were numbered and annotated according to the Kabat numbering system.
The humanized light chain templates of murine antibody mAb-001 are IGKV1-39*01 and hjk2.1, and the humanized heavy chain templates are IGHV1-2*02 and hjh2. The variable region sequence of humanized antibody h001-1 obtained after humanization is as follows:
> h001-1 VH 
QVQLVQSGAEVKKPGASVKVSCKASGYTFTDYWMHWVRQAPGQGLEWMGYIN
PSSGFTKYHQNFKDRVTMTRDTSISTAYMELSRLRSDDTAVYYCARQYDYDEDW
YFDVWGQGTTVTVSS SEQ ID NO: 18
> h001-1 VL 
DIQMTQSPSSLSASVGDRVTITCKSSQSLLNSRTRKNFLAWYQQKPGKAPKLLIY
WASTRESGVPSRFSGSGSGTDFTLTISSLQPEDFATYYCKQSFNLFTFGQGTKLEIK
SEQ ID NO: 24
Note: The order of variable region is FR1-CDR1-FR2-CDR2-FR3-CDR3-FR4, the italic is FR sequence, and the underlined is CDR sequence.
2. Template selection and back mutation design for hybridoma clone mAb-001 are shown in Table 2. The combination of humanized sequence after back mutation of hybridoma is shown in Table 2.

**Table 2: Template selection and back mutation design**

| VH | | SEQ ID NO | VL | | SEQ ID NO |
|---|---|---|---|---|---|
| h001_VH.1 | Grafted | 18 | h001_VL.1 | Grafted | 24 |
| h001_VH.1A | T30N | 19 | h001_VL.1A | S66D | 25 |
| h001_VH.1B | R87T | 20 | h001_VL.1B | T5S, S66D | 26 |
| h001_VH.1C | T30N, R87T | 21 | h001_VL.1C | T5S, S66D, Q3V, A49S | 27 |
| h001_VH.1D | T30N, R87T, R72A, T74K | 22 | | | |
| h001_VH.1E | T30N, R87T, R72A, T74K, M48I, V68A, | 23 | | | |
| | M70L, R38K, R67K | | | | |

Note: For example, according to Kabat numbering system, S66D means S at position 66 was back-mutated to D.
"Grafted" represents that murine antibody CDRs were grafted into human germline FR region sequences, and the sepcific sequences of the mutant variable regions are shown in Table 3:

**Table 3: Variable region sequences of each mutant**

| SEQ ID NO | Sequence |
|---|---|
| 19 | QVQLVQSGAEVKKPGASVKVSCKASGYTFNDYWMHWVRQAPGQGLEW MGYINPSSGFTKYHQNFKDRVTMTRDTSISTAYMELSRLRSDDTAVYYCAR QYDYDEDWYFDVWGQGTTVTVSS |
| 20 | QVQLVQSGAEVKKPGASVKVSCKASGYTFTDYWMHWVRQAPGQGLEW MGYINPSSGFTKYHQNFKDRVTMTRDTSISTAYMELSRLTSDDTAVYYCAR QYDYDEDWYFDVWGQGTTVTVSS |
| 21 | QVQLVQSGAEVKKPGASVKVSCKASGYTFNDYWMHWVRQAPGQGLEW MGYINPSSGFTKYHQNFKDRVTMTRDTSISTAYMELSRLTSDDTAVYYCAR QYDYDEDWYFDVWGQGTTVTVSS |
| 22 | QVQLVQSGAEVKKPGASVKVSCKASGYTFNDYWMHWVRQAPGQGLEW MGYINPSSGFTKYHQNFKDRVTMTADKSISTAYMELSRLTSDDTAVYYCAR QYDYDEDWYFDVWGQGTTVTVSS |
| 23 | QVQLVQSGAEVKKPGASVKVSCKASGYTFNDYWMHWVKQAPGQGLEWI GYINPSSGFTKYHQNFKDKATLTADKSISTAYMELSRLTSDDTAVYYCARQ YDYDEDWYFDVWGQGTTVTVSS |
| 25 | DIQMTQSPSSLSASVGDRVTITCKSSQSLLNSRTRKNFLAWYQQKPGKAP KLLIYWASTRESGVPDRFSGSGSGTDFTLTISSLQPEDFATYYCKQSFNLF TFGQGTKLEIK |
| 26 | DIQMSQSPSSLSASVGDRVTITCKSSQSLLNSRTRKNFLAWYQQKPGKAP KLLIYWASTRESGVPDRFSGSGSGTDFTLTISSLQPEDFATYYCKQSFNLF TFGQGTKLEIK |
| 27 | DIVMSQSPSSLSASVGDRVTITCKSSQSLLNSRTRKNFLAWYQQKPGKSP KLLIYWASTRESGVPDRFSGSGSGTDFTLTISSLQPEDFATYYCKQSFNLF TFGQGTKLEIK |

Note: The underlined parts of sequences are CDR regions.

**Table 4: Humanized sequence combination of murine antibody mAb-001**

| | h001_VL.1 | h001_VL.1A | h001_VL.1B | h001_VL.1C |
|---|---|---|---|---|
| h001_VH.1 | h001-1 | h001-2 | h001-3 | h001-4 |
| h001_VH.1A | h001-5 | h001-6 | h001-7 | h001-8 |
| h001_VH.1B | h001-9 | h001-10 | h001-11 | h001-12 |
| h001_VH.1C | h001-13 | h001-14 | h001-15 | h001-16 |
| h001_VH.1D | h001-17 | h001-18 | h001-19 | h001-20 |
| h001_VH.1E | h001-21 | h001-22 | h001-23 | h001-24 |

Note: The combination of humanized antibody variable region obtained by combining various sequences and mutated sequences thereof is shown in the above Table. For example, h001-1 indicates that the variable region of humanized antibody h001-1 consists of light chain h001_VL1 and heavy chain h001 VH.1A, and so on.
3. The above humanized sequences were combined to form an antibody, wherein the heavy chain constant region is derived from human IgG1, and the light chain constant region is derived from human kappa chain. The corresponding humanized antibody was obtained, and it was verified that the PCSK9 antibodies obtained in the present invention have relatively high binding activity with PCSK9 and PCSK9-Y. Also, the antibodies can effectively block the binding of PCSK9/PCSK9-Y to LDLR.

### Example 5. Construction and Expression of Anti-human PCSK9 Humanized Antibodies IgG1 and IgGl-YTE Formats.

The method for constructing and expressing anti-human PCSK9 humanized antibodies is as follows:
1. Primer design: Multiple primers were designed by using the online software DNAWorks (v3.2.2, http://helixweb.nih.gov/dnaworks/) to synthesize VH/VK containing gene fragments necessary for recombination: 5' -30bp Signal peptide + VH/VK + 30bp CH1/CL- 3'. The principle of primer design is as follows: if the target gene 2 differs from the target gene 1 in 2 amino acids, another primer specific for the mutation site was designed, as shown in Figure 1.
2. Fragment splicing: according to the Manuals for TakaRa Primer STAR GXL DNA polymerase, two-step PCR amplification was performed with the multiple primers designed above and VH/VK containing gene fragments necessary for recombination was obtained.
3. Construction of expression vector pHr (with signal peptide and constant region gene (CH1-FC/CL) fragment) and restriction enzyme digestion.
   Expression vector pHr (with signal peptide and constant region gene (CH1-FC/CL) fragment) were designed and constructed by utilizing the properties of some special restriction enzymes, such as BsmBI, whose recognition sequence is different from its cleavage site, as shown in Figure 2. BsmBI was used to linearize the vector, and the gel was cut and recovered for later use.
4. Construction of the recombinant expression vector VH-CH1-FC -pHr/VK-CL-pHr.

VH/VK containing the gene fragments necessary for recombination and the recovered expression vector pHr (with the signal peptide and the constant region gene (CH1-FC/CL) fragment) digested with BsmBI enzyme were added into the DH5 alpha competent cells at a ratio of 3:1, incubated in an ice bath at 0°C for 30 min, and heat shocked for 90 second at 42°C. Then 5 times volume of LB medium was added, incubated at 37°C for 45 min, plated on LB-Amp plate, and cultured at 37°C overnight. A single clone was picked and sequenced to obtain the target clone.

The antibody of this invention can be designed and constructed according to, but is not limited to, the above method. Taking h001-4 as an example, the antibody and variants thereof were designed to obtain: 1) h001-4-WT: an IgG format of h001-4, i.e., humanized sequence combination h001-4, combining heavy chain constant region derived from human IgG1 with light chain constant region derived from human kappa chain; 2) h001-4-YTE: h001-4-IgG1-YTE format, i.e., humanized sequence combination h001-4, combining heavy chain constant region of mutant human IgG1 (YTE mutation) with light chain constant region derived from human kappa chain. Mutated human IgG1 may also be other forms of mutation.

### The Sequences of Constructed and Expressed Anti-human PCSK9 Humanized Antibodies (IgGl and IgGl-YTE Formats thereof) are as follows:

H001-4 IgG1 format, its heavy chain constant region is from human IgG1 and light chain constant region is from human kappa light chain.
Amino acid sequence of heavy chain (Human IgG1) is as follows: 
QVQLVQSGAEVKKPGASVKVSCKASGYTFTDYWMHWVRQAPGQGLEWMG
YINPSSGFTKYHQNFKDRVTMTRDTSISTAYMELSRLRSDDTAVYYCARQYDY
DEDWYFDVWGQGTTVTVSSASTKGPSVFPLAPSSKSTSGGTAALGCLVKDYF
PEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPE
VTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDK
SRWQQGNVFSCSVMHEALHNHYTQKSLSLSPGK
SEQ ID NO: 28
DNA sequence of heavy chain is as follows: ATGGAGTTTGGGCTGAGCTGGCTTTTTCTTGTCGCGATTCTTAAGGGTGTCC 
AGTGCCAGGTGCAGCTGGTGCAGAGCGGCGCTGAGGTGAAGAAGCCCGG
AGCGAGCGTAAAGGTGAGCTGCAAGGCCAGCGGATACACCTTCACCGACT
ACTGGATGCACTGGGTGAGGCAGGCCCCAGGACAGGGCCTGGAGTGGATG
GGCTACATCAACCCCAGCAGCGGCTTTACCAAGTATCACCAGAACTTCAAA
GACAGGGTGACCATGACCAGGGACACCAGCATCAGCACCGCCTACATGGA
GCTGAGCAGGCTGAGGAGCGACGACACCGCCGTGTACTACTGCGCCAGGC
AATACGACTACGACGAGGACTGGTACTTCGACGTGTGGGGCCAAGGAACC
ACCGTGACTGTGAGCAGCGCTTCGACCAAGGGCCCATCGGTCTTCCCCCTG
GCACCCTCCTCCAAGAGCACCTCTGGGGGCACAGCGGCCCTGGGCTGCCT
GGTCAAGGACTACTTCCCCGAACCGGTGACGGTGTCGTGGAACTCAGGCG
CCCTGACCAGCGGCGTGCACACCTTCCCGGCTGTCCTACAGTCCTCAGGAC
TCTACTCCCTCAGCAGCGTGGTGACCGTGCCCTCCAGCAGCTTGGGCACCC
AGACCTACATCTGCAACGTGAATCACAAGCCCAGCAACACCAAGGTGGAC
AAGAAAGTTGAGCCCAAATCTTGTGACAAAACTCACACATGCCCACCGTG
CCCAGCACCTGAACTCCTGGGGGGACCGTCAGTCTTCCTCTTCCCCCCAAA
ACCCAAGGACACCCTCATGATCTCCCGGACCCCTGAGGTCACATGCGTGGT
GGTGGACGTGAGCCACGAAGACCCTGAGGTCAAGTTCAACTGGTACGTGG
ACGGCGTGGAGGTGCATAATGCCAAGACAAAGCCGCGGGAGGAGCAGTAC
AACAGCACGTACCGTGTGGTCAGCGTCCTCACCGTCCTGCACCAGGACTG
GCTGAATGGCAAGGAGTACAAGTGCAAGGTCTCCAACAAAGCCCTCCCAG
CCCCCATCGAGAAAACCATCTCCAAAGCCAAAGGGCAGCCCCGAGAACCA
CAGGTGTACACCCTGCCCCCATCCCGGGATGAGCTGACCAAGAACCAGGT
CAGCCTGACCTGCCTGGTCAAAGGCTTCTATCCCAGCGACATCGCCGTGGA
GTGGGAGAGCAATGGGCAGCCGGAGAACAACTACAAGACCACGCCTCCC
GTGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGA
GGCTCTGCACAACCACTACACGCAGAAGAGCCTCTCCCTGTCTCCGGGTA
AATGA
SEQ ID NO: 29
 h001-4-kappa
Amino acid sequence of light chain is as follows: 
DIVMSQSPSSLSASVGDRVTITCKSSQSLLNSRTRKNFLAWYQQKPGKSPKLLI
YWASTRESGVPDRFSGSGSGTDFTLTISSLQPEDFATYYCKQSFNLFTFGQGTK
LEIKRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSF
NRGEC
SEQ ID NO: 30
DNA sequence of light chain is as follows: 
ATGGACATGCGCGTGCCCGCCCAGCTGCTGGGCCTGCTGCTGCTGTGGTTC
CCCGGCTCGCGATGCGACATCGTGATGTCTCAGAGCCCATCTAGCCTGAGC
GCCAGCGTGGGCGACAGGGTAACCATCACCTGCAAGAGCAGCCAAAGCCT
GCTGAACAGCAGGACCCGCAAGAACTTCCTGGCTTGGTATCAGCAGAAGC
CCGGCAAGTCTCCCAAGTTGCTGATCTACTGGGCCAGCACCAGGGAGAGC
GGCGTGCCCGACAGGTTCAGCGGCTCCGGCAGCGGCACCGACTTCACCCT
GACCATCTCTAGTCTGCAGCCCGAGGACTTCGCCACCTACTACTGCAAGCA
GAGCTTCAATCTGTTCACCTTCGGCCAGGGCACCAAGCTGGAGATCAAGC
GTACGGTGGCTGCACCATCTGTCTTCATCTTCCCGCCATCTGATGAGCAGTT
GAAATCTGGAACTGCCTCTGTTGTGTGCCTGCTGAATAACTTCTATCCCAGA
GAGGCCAAAGTACAGTGGAAGGTGGATAACGCCCTCCAATCGGGTAACTC
CCAGGAGAGTGTCACAGAGCAGGACAGCAAGGACAGCACCTACAGCCTC
AGCAGCACCCTGACGCTGAGCAAAGCAGACTACGAGAAACACAAAGTCT
ACGCCTGCGAAGTCACCCATCAGGGCCTGAGCTCGCCCGTCACAAAGAGC
TTCAACAGGGGAGAGTGTTGA
SEQ ID NO: 31
 Light chain of h001-4-IgG1-YTE is h001-4-kappa: SEQ ID NO: 30)
Amino acid sequence of heavy chain of IgG1-YTE is as follows: 
QVQLVQSGAEVKKPGASVKVSCKASGYTFTDYWMHWVRQAPGQGLEWMG
YINPSSGFTKYHQNFKDRVTMTRDTSISTAYMELSRLRSDDTAVYYCARQYDY
 
DEDWYFDVWGQGTTVTVSSASTKGPSVFPLAPSSKSTSGGTAALGCLVKDYF
PEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLYITREPE
VTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDK
SRWQQGNVFSCSVMHEALHNHYTQKSLSLSPGK
SEQ ID NO: 32
DNA sequence of heavy chain is as follows: 
ATGGAGTTTGGGCTGAGCTGGCTTTTTCTTGTCGCGATTCTTAAGGGTGTCC
AGTGCCAGGTGCAGCTGGTGCAGAGCGGCGCTGAGGTGAAGAAGCCCGG
AGCGAGCGTAAAGGTGAGCTGCAAGGCCAGCGGATACACCTTCACCGACT
ACTGGATGCACTGGGTGAGGCAGGCCCCAGGACAGGGCCTGGAGTGGATG
GGCTACATCAACCCCAGCAGCGGCTTTACCAAGTATCACCAGAACTTCAAA
GACAGGGTGACCATGACCAGGGACACCAGCATCAGCACCGCCTACATGGA
GCTGAGCAGGCTGAGGAGCGACGACACCGCCGTGTACTACTGCGCCAGGC
AATACGACTACGACGAGGACTGGTACTTCGACGTGTGGGGCCAAGGAACC
ACCGTGACTGTGAGCAGCGCTTCGACCAAGGGCCCATCGGTCTTCCCCCTG
GCACCCTCCTCCAAGAGCACCTCTGGGGGCACAGCGGCCCTGGGCTGCCT
GGTCAAGGACTACTTCCCCGAACCGGTGACGGTGTCGTGGAACTCAGGCG
CCCTGACCAGCGGCGTGCACACCTTCCCGGCTGTCCTACAGTCCTCAGGAC
TCTACTCCCTCAGCAGCGTGGTGACCGTGCCCTCCAGCAGCTTGGGCACCC
AGACCTACATCTGCAACGTGAATCACAAGCCCAGCAACACCAAGGTGGAC
AAGAAAGTTGAGCCCAAATCTTGTGACAAAACTCACACATGCCCACCGTG
CCCAGCACCTGAACTCCTGGGGGGACCGTCAGTCTTCCTCTTCCCCCCAAA
ACCCAAGGACACCCTCTACATCACCCGGGAGCCTGAGGTCACATGCGTGG
TGGTGGACGTGAGCCACGAAGACCCTGAGGTCAAGTTCAACTGGTACGTG
GACGGCGTGGAGGTGCATAATGCCAAGACAAAGCCGCGGGAGGAGCAGT
ACAACAGCACGTACCGTGTGGTCAGCGTCCTCACCGTCCTGCACCAGGAC
TGGCTGAATGGCAAGGAGTACAAGTGCAAGGTCTCCAACAAAGCCCTCCC
 
AGCCCCCATCGAGAAAACCATCTCCAAAGCCAAAGGGCAGCCCCGAGAAC
CACAGGTGTACACCCTGCCCCCATCCCGGGATGAGCTGACCAAGAACCAG
GTCAGCCTGACCTGCCTGGTCAAAGGCTTCTATCCCAGCGACATCGCCGTG
GAGTGGGAGAGCAATGGGCAGCCGGAGAACAACTACAAGACCACGCCTC
CCGTGCTGGACTCCGACGGCTCCTTCTTCCTCTACAGCAAGCTCACCGTGG
ACAAGAGCAGGTGGCAGCAGGGGAACGTCTTCTCATGCTCCGTGATGCAT
GAGGCTCTGCACAACCACTACACGCAGAAGAGCCTCTCCCTGTCTCCGGG
TAAATGA
SEQ ID NO: 33
Note: The underlined part is DNA sequence of signal peptide.

### Exemplary preparation process of antibody pharmaceutical composition (formulation)

Step1: A certain amount of purified anti-PCSK-9 antibody sample (such as h001-4-YTE) was taken, and the solvent (preferably by ultrafiltration) was replaced with 6 times the volume of antibody-free buffer (such as 10 mM, pH 6.0, histidine-hydrochloride buffer) by ultrafiltration membrane until the protein was concentrated to 60 mg/mL (±2 mg/mL). A certain volume of sucrose stock solution was added and mixed until the final sucrose concentration was 25 mg/mL. A certain volume of Tween-80 stock solution was added and mixed until the final Tween-80 concentration was 0.2 mg/mL. 10 mM, pH 6.0, histidine buffer was added to finalize the protein at a concentration of 50 mg/mL (±5mg/mL). The pH of final product is about 6.3 ± 0.1 (other formulations to be tested or stable formulations shall be prepared by similar procedures).

After the product was filtered, samples of the in-process control was taken to determine its sterility. The drug substance was passed through a 0.22 µm PVDF filter element and the filtrate was collected.

Step 2: The loading volume was adjusted to 3.6 mL, the filtrate was filled in 6 mL vial with a partial stopper, the loading difference was detected by in-process control sampling at the beginning, the middle and the end of the filling, respectively.

Step 3: The solution with a stopper was filled into the lyophilization chamber and lyophilized. The lyophilization process includes pre-freezing, primary drying and secondary drying. After the lyophilization procedure was completed, the lyophilized powder was plugged in a vacuum.

| Parameter of lyophilization process | Temperature(°C) | Vacuum degree (mBar) |
|---|---|---|
| Pre-freezing | 5 | / |
| | -45 | / |
| Primary drying | -5 | 0.3 |
| Secondary drying | 25 | 0.3 |
| | 25 | 0.01 |

The time used for lyophilization can be adjusted according to the actual situation. The type of lyophilizer, the loading capacity of lyophilized pharmacy, and the container of lyophilized medicament will affect the lyophilization time. Such adjustments in time are well known to those skilled in the art.

Step 4: The capping machine was turned on, aluminum cover was added, thus conducting capping.

Step 5: Visual inspection was performed to confirm the product is free from defects such as collapse, incorrect loading capacity. The label of the vial was printed and pasted; the label of carton was printed and the carton was folded, packaged and the label of the box was pasted.

### Example 6. Screening of Buffer System

Anti-PCSK9 antibody formulation samples with a protein concentration of 1 mg/ml were prepared with the following buffer solutions.
1) 20 mM citric acid-sodium citrate, pH 4.0
2) 20 mM citric acid-sodium citrate, pH 4.5
3) 20 mM citric acid-sodium citrate, pH 5.0
4) 20 mM citric acid-sodium citrate, pH 5.5
5) 20 mM citric acid-sodium citrate, pH 6.0
6) 20 mM sodium dihydrogen phosphate-disodium hydrogen phosphate, pH 6.0
7) 20 mM sodium dihydrogen phosphate-disodium hydrogen phosphate, pH 6.5
8) 20 mM sodium dihydrogen phosphate-disodium hydrogen phosphate, pH 7.0
9) 20 mM sodium dihydrogen phosphate-disodium hydrogen phosphate, pH 7.5
10) 20 mM Tris-HCl, pH 7.5
11) 20 mM Tris-HCl, pH 8.0
12) 20 mM Tris-HCl, pH 8.5

The thermal stability of the anti-PCSK9 antibody in each formulation sample was determined by differential scanning calorimetry (DSC). The thermal denaturation midpoint temperature (Tm) analysis of the anti-PCSK-9 antibody showed that it had better thermal stability at pH ≥ 6.0. The results are shown in Table 5. Therefore, buffers with pH between 6.0 and 6.5, such as histidine-hydrochloride, sodium dihydrogen phosphate-disodium hydrogen phosphate, sodium succinate-succinate, and citric acid-sodium citrate were selected for subsequent studies.

**Table 5. DSC screening results of H001-4-YTE in different buffer system and pH value**

| H001-4-YTE (mg/ml) | sucrose (mg/ml) | pH | Buffer system | Tmₒₙₛₑₜ (°C) | Tm (°C) |
|---|---|---|---|---|---|
| 1 | N/A | 4.0 | 20 mM citric acid-sodium citrate | 39.17 | 66.02 |
| | | 4.5 | 20 mM citric acid-sodium citrate | 42.34 | 68.52 |
| | | 5.0 | 20 mM citric acid-sodium citrate | 44.25 | 69.77 |
| | | 5.5 | 20 mM citric acid-sodium citrate | 49.5 | 70.19 |
| | | 6.0 | 20 mM citric acid-sodium citrate | 53.57 | 70.75 |
| | | 6.0 | 20 mM sodium dihydrogen phosphate-di sodium hydrogen phosphate | 54.79 | 72.12 |
| | | 6.5 | 20mM sodium dihydrogen phosphate-disodium hydrogen phosphate | 52.2 | 72.54 |
| | | 7.0 | 20mM sodium dihydrogen phosphate-disodium hydrogen phosphate | 50.84 | 73.19 |
| | | 7.5 | 20mM sodium dihydrogen phosphate-disodium hydrogen phosphate | 50.64 | 73.98 |
| | | 7.5 | 20mM Tris-HCl | 47.36 | 72.54 |
| | | 8.0 | 20mM Tris-HCl | 49.09 | 73.43 |
| | | 8.5 | 20mM Tris-HCl | 52.47 | 74.13 |

| | | | | | |
|---|---|---|---|---|---|
| Note: N/A represents that the ingredient is not added. | | | | | |

Anti-PCSK9 antibody formulation samples with a protein concentration of 1 mg/ml were prepared with the following buffer solutions.
1) 20 mM histidine-hydrochloride, pH 6.0
2) 20 mM histidine-hydrochloride, pH 6.5
3) 20 mM sodium dihydrogen phosphate-disodium hydrogen phosphate, pH 6.0
4) 20 mM sodium dihydrogen phosphate-disodium hydrogen phosphate, pH 6.5
5) 20 mM succinic acid- sodium succinate, pH 6.0
6) 20 mM citric acid-sodium citrate, pH 6.0
7) 20 mM citric acid-sodium citrate, pH 6.5

The thermal stability of anti-PCSK9 antibody in each formulation sample was determined by differential scanning calorimetry (DSC). The thermal denaturation midpoint temperature (Tm) analysis of the anti-PCSK-9 antibody showed (see Table 6) that the anti-PCSK-9 antibody was slightly more stable in the histidine, phosphate and succinate buffer systems than that in the citrate buffer system.

**Table 6. DSC screening results of H001-4-YTE in different buffer system and pH value**

| H001-4-YTE (mg/ml) | sucrose (mg/ml) | pH | Buffer system | Tm₁ (°C) | Tm (°C) |
|---|---|---|---|---|---|
| 1 | N/A | 6.0 | 20mM histidine-hydrochloride | 63.51 | 72.01 |
| | | 6.5 | 20mM histidine-hydrochloride | 65.34 | 72.45 |
| | | 6.0 | 20mM sodium dihydrogen phosphate-di sodium hydrogen phosphate | 64.74 | 72.18 |
| | | 6.5 | 20mM sodium dihydrogen phosphate-disodium hydrogen phosphate | 65.2 | 72.61 |
| | | 6.0 | 20 mM sodium succinate-succinate | 64.31 | 71.87 |
| | | 6.0 | 20 mM citric acid-sodium citrate | 63.73 | 70.72 |
| | | 6.5 | 20 mM citric acid-sodium citrate | 64.46 | 71.42 |

| | | | | | |
|---|---|---|---|---|---|
| Note: N/A represents that the ingredient is not added. | | | | | |

### Example 7. Screening of Saccharide in formulation

Anti-PCSK9 antibody formulation samples with a protein concentration of 150 mg/ml were prepared with the following buffer solutions.
1) 20 mM histidine-hydrochloride, 70 mg/ml sucrose, pH 6.5
2) 20 mM histidine-hydrochloride, 70 mg/ml α, α-dihydrate trehalose, pH 6.5

Each formulation was filtered and filled into a 2 mL vial at 0.5 mL/bottle, and the vial was sealed with a stopper. Samples were taken and subjected to 40 °C high temperature experiment, low temperature illumination experiment and freeze-thaw cycle experiment (samples were placed at -20 ° C for 24 h and then taken out and placed at room temperature to completely melt and mix the sample, which is a cycle). The results showed that the sucrose and trehalose have similar effects on the stability of anti-PCSK-9 antibody. Sucrose was selected as a stabilizer for anti-PCSK-9 antibody, and the results are shown in Table 7.

**Table 7. Accelerated experimental results of different saccharides**

| No. | Time | Appearance | SEC Monomer% | IEC Acid Peak% | IEC Main Peak% |
|---|---|---|---|---|---|
| 1 | Day 0 | Clear and transparent | 99.67 | 21.87 | 60.79 |
| | 40 °C Day 21 | Clear and transparent | 96.96 | 39.09 | 27.92 |
| | Freezing and thawing 5 times | Clear and transparent | 99.83 | 21.17 | 61.32 |
| | 2-8°C Illumination Day 10 | Clear and transparent | 98.62 | 47.25 | 33.44 |
| 2 | Day 0 | Clear and transparent | 99.93 | 22.57 | 59.38 |
| | 40 °C Day 21 | Clear and transparent | 97.19 | 38.17 | 27.73 |
| | Freezing and thawing 5 times | Clear and transparent | 99.73 | 21.03 | 61.42 |
| | 2-8°C Illumination Day 10 | Clear and transparent | 98.02 | 50.05 | 30.87 |

Anti-PCSK9 antibody formulation samples with a protein concentration of 150 mg/ml were prepared with the following buffer solutions.
1) 20 mM histidine-hydrochloride, 0 mg/ml sucrose, pH 6.5
2) 20 mM histidine-hydrochloride, 10 mg/ml sucrose, pH 6.5
3) 20 mM histidine-hydrochloride, 40 mg/ml sucrose, pH 6.5
4) 20 mM histidine-hydrochloride, 70 mg/ml sucrose, pH 6.5

The osmotic pressure value indicates that the osmotic pressure meets the minimum subcutaneous injection requirement when the concentration of sucrose is ≥ 70 mg/ml. The anti-PCSK-9 antibody formulation of the invention is prepared by a process of lyophilization in low concentration and reconstitution in high concentration (Three times the volume of the drug substance was lyophilized, one volume of water for injection was reconstituted, and the present invention adopts the above ratio to perform freeze-reconstitution unless specified otherwise). When the sucrose concentration in the final prescription (reconstituted formulation) is set at 75 mg/ml, it not only satisfies the osmotic pressure requirement for injection, but also facilitates the setting of the sucrose concentration in the drug substance, and the sucrose concentration of 25 mg/ml is easy to freeze-dry.

**Table 8. Determination Results of Sucrose concentration-osmotic pressure of H001-4-YTE formulation**

| H001-4-YTE (mg/ml) | Saccharide | Saccharide concentration(mg/ml) | Osmotic pressure(mosm) |
|---|---|---|---|
| 150 | N/A | 0 | 44 |
| | sucrose | 10 | 71 |
| | | 40 | 153 |
| | | 70 | 290 |

| | | | |
|---|---|---|---|
| Note: N/A represents that the ingredient is not added. | | | |

### Example 8. Screening of surfactants in formulations

Anti-PCSK9 antibody formulation samples with a protein concentration of 150 mg/ml were prepared with the following buffer solutions.
1) 20 mM histidine-hydrochloride, pH 6.5
2) 20 mM histidine-hydrochloride, 0.2 mg/ml polysorbate 20, pH 6.5
3) 20 mM histidine-hydrochloride, 0.4 mg/ml polysorbate 80, pH 6.5

Each formulation sample was filtered and filled into a 2 mL vial at 0.5 mL/bottle, and the vial was sealed with a stopper. Samples were placed on a 25 °C constant temperature shaker and shaken at 300 rpm. Appearance results indicated that surfactants effectively prevent aggregation of anti-PCSK-9 antibodies. There was no significant difference between polysorbate 20 and polysorbate 80 on H001-4-YTE. Polysorbate 80 was selected as the stabilizer of anti-PCSK-9 antibody formulation.

**Table 9. Effect of Surfactant on H001-4-YTE Aggregation shaken at 300 rpm, 25°C**

| No. | Time | Appearance | SEC Monomer% | IEC Acid Peak % | IEC Main Peak% |
|---|---|---|---|---|---|
| 1 | Day 0 | Clear and | 99.72 | 21.98 | 60.60 |
| | | transparent | | | |
| | Day 7 | Large amount of particles, turbid | 99.88 | 20.99 | 61.63 |
| 2 | Day 0 | Clear and transparent | 99.73 | 23.35 | 58.09 |
| | Day 7 | Clear and transparent | 99.78 | 21.71 | 59.43 |
| 3 | Day 0 | Clear and transparent | 99.76 | 23.22 | 58.33 |
| | Day 7 | Clear and transparent | 99.74 | 21.83 | 59.42 |

### Example 9. Screening and confirmation of buffer system in formulation

Formulation samples containing 25 mg/ml sucrose, 0.2 mg/ml polysorbate 80 and 50 mg/ml antibody was prepared using buffer solution containing 20 mM histidine-hydrochloride or 20 mM succinic acid-sodium succinate at pH 6.0 or 6.5. Each formulation was filtered and filled into a 6 mL vial at 3.6 mL/vial for lyophilization, and the vial was sealed with a halogenated butyl rubber stopper which was used for freeze-dried sterile powder. The lyophilized product was stored at 2-8 °C, 25 °C and 40°C, and then reconstituted with injection water for stability analysis. The results showed that the anti-PCSK9 antibody was very stable at 2-8 °C and 25 °C. However, the appearance of reconstituted formulation on day 0 showed that the succinic acid buffer at pH 6.0 had significant opalescence compared to the His system (not shown in the table), so the histidine system was selected as a buffer system for the anti-PCSK-9 antibody.

**Table 10. Stability of H001-4-YTE lyophilized powder at different temperatures**

| Buffer and pH | Temperature and Time | SEC Main peak | IEC Neutral peak | CE Non-reducing |
|---|---|---|---|---|
| Succinate buffer pH6.0 | 0 point | 99.5 | 57.5 | 96.4 |
| | 40°C, 1 month | 97.7 | 50.5 | 96.2 |
| | 25°C, 6 months | 98.9 | 57.5 | 94.0 |
| | 2-8 °C, 9 momths | 99.0 | 59.5 | 95.4 |
| Histidine buffer pH6.0 | 0 point | 99.6 | 54.5 | 96.9 |
| | 40°C, 1 month | 98.0 | 51.1 | 96.2 |
| | 25°C, 6 months | 98.7 | 56.3 | 95.1 |
| | 2-8 °C, 9momths | 99.3 | 58.8 | 95.1 |
| Histidine buffer pH6.5 | 0 point | 99.7 | 57.4 | 96.4 |
| | 40°C, 1 month | 97.5 | 52.7 | 96.0 |
| | 25°C, 6 months | 98.6 | 55.1 | 93.2 |
| | 2-8 °C, 9momths | 98.3 | 58.9 | 94.9 |

### Example 10. Comprehensive screening of formulation components

The anti-PCSK-9 antibody formulation samples containing 50 mg/ml anti-PCSK-9 and 25 mg/ml sucrose were prepared with the following buffers at different pH, different ionic strength and containing different concentrations of surfactants.
1) 10 mM histidine-hydrochloride, 0.1 mg/ml polysorbate 80, pH 6.5
2) 10 mM histidine-hydrochloride, 0.3 mg/ml polysorbate 80, pH 6.0
3) 10 mM histidine-hydrochloride, 0.2 mg/ml polysorbate 80, pH 5.5
4) 15 mM histidine-hydrochloride, 0.3 mg/ml polysorbate 80, pH 5.5
5) 10 mM histidine-hydrochloride, 0.2 mg/ml polysorbate 80, pH 6.0
6) 15 mM histidine-hydrochloride, 0.3 mg/ml polysorbate 80, pH 6.5
7) 15 mM histidine-hydrochloride, 0.2 mg/ml polysorbate 80, pH 6.0
8) 5 mM histidine-hydrochloride, 0.3 mg/ml polysorbate 80, pH 5.5
9) 15 mM histidine-hydrochloride, 0.1 mg/ml polysorbate 80, pH 5.5
10) 5 mM histidine-hydrochloride, 0.1 mg/ml polysorbate 80, pH 6.0
11) 5 mM histidine-hydrochloride, 0.2 mg/ml polysorbate 80, pH 6.5

Each formulation sample was filtered and filled into a 6 mL vial at 3.6 mL/bottle for lyophilization, and the vial was sealed with a halogenated butyl rubber stopper which was used for lyophilized sterile powder. The lyophilized product was stored at 40 °C for stability analysis. Data analysis based on the difference in SEC monomer showed that the formulation of anti-PCSK-9 antibody using 10 mM histidine-hydrochloride buffer with pH 6.0 containing 0.2 mg/ml polysorbide 80 was more stable under the condition of 50 mg/ml protein and 25 mg/ml sucrose. After lyophilization and reconstitution, the concentration of each component in the final formulation (150 mg/ml protein, 75 mg/ml sucrose, 30 mM histidine-hydrochloride buffer, and 0.6 mg/ml polysorbate 80) was determined as three times concentration of the drug substance before lyophilization, pH 6.3 ± 0.1.

**Table 11. Experiment results of H001-4-YTE at high temperature**

| No. | Time | Appearance | SEC Monomer % | CE |
|---|---|---|---|---|
| 1 | Day 0 | Clear and transparent | 98.65 | 95.70 |
| | Day 24 | Clear and transparent | 97.98 | 96.23 |
| 2 | Day 0 | Clear and transparent | 98.76 | 95.65 |
| | Day 24 | Clear and transparent | 98.70 | 96.26 |
| 3 | Day 0 | Clear and transparent | 98.78 | 95.75 |
| | Day 24 | Clear and transparent | 97.73 | 96.34 |
| 4 | Day 0 | Clear and transparent | 98.73 | 95.66 |
| | Day 24 | Clear and transparent | 97.64 | 96.14 |
| 5 | Day 0 | Clear and transparent | 98.74 | 95.70 |
| | Day 24 | Clear and transparent | 97.53 | 96.30 |
| 6 | Day 0 | Clear and transparent | 98.72 | 95.74 |
| | Day 24 | Clear and transparent | 97.49 | 96.10 |
| 7 | Day 0 | Clear and transparent | 98.75 | 95.77 |
| | Day 24 | Clear and transparent | 97.50 | 96.11 |
| 8 | Day 0 | Clear and transparent | 98.70 | 95.75 |
| | Day 24 | Clear and transparent | 97.32 | 95.89 |
| 9 | Day 0 | Clear and transparent | 98.37 | 95.78 |
| | Day 24 | Clear and transparent | 97.68 | 96.19 |
| 10 | Day 0 | Clear and transparent | 98.80 | 95.73 |
| | Day 24 | Clear and transparent | 97.26 | 96.08 |
| 11 | Day 0 | Clear and transparent | 98.44 | 95.67 |
| | Day 24 | Clear and transparent | 97.06 | 96.01 |

### Example 11. Temperature optimization of the primary drying

Anti-PCSK9 antibody formulation samples with an anti-PCSK9 antibody concentration of 50mg/ml, 25 mg/ml sucrose, 0.2 mg/ml polysorbate 80 were prepared with the 10 mM histidine-hydrochloride at pH 6.0. The antibody was filled into a 6 mL vial with 3.6 mL/vial and lyophilized at a primary drying temperature of -14°C, and - 5 °C respectively, and the vial was sealed with a stopper for lyophilization. The reconstituted samples before and after lyophilization were compared. The results show that -5 °C is the preferred primary drying temperature of the lyophilization process.

**Table12. Comparison of H001-4-YTE samples prepared with different drying processes before and after freeze-drying**

| Test items | Before freeze-drying | Lyophilized at - 14°C, and Reconstituted | Lyophilized at - 5°C, and Reconstituted |
|---|---|---|---|
| Concentration | 52.3mg/ml | 152.5mg/ml | 148.0 mg/ml |
| pH | 6.32 | 6.36 | 6.32 |
| Appearance | Clear and transparent | Clear and transparent | Clear and transparent |
| SEC Monomer (%) | 99.8 | 99.8 | 99.9 |
| CE Non-reducing (%) | 96.3 | 96.2 | 96.3 |
| IEC Main Peak (%) | 61.3 | 61.4 | 60.5 |
| Moisture content | N/A | 0.5% | 0.6% |

### Example 12. Determination of compatibility between formulation and containers of different materials

H001-4-YTE was prepared at 50mg/mL in a solution comprising 10 mM histidine-hydrochloride, pH 6.0, with 25 mg/ml sucrose and 0.2 mg/ml polysorbate 80. The formulation samples were filled in glass bottles, liquid storage bags and 316 L stainless steel tanks respectively, and placed at 2-8 °C for 24 hours. Protein content and purity analysis showed that H001-4-YTE was stable within 24 hours. The formulation was compatible with the 316 L stainless steel tank, glass bottle and liquid storage bag.

**Table 13. Stability of H001-4-YTE in different contact materials**

| Materi al | Temperat ure | Tim e | SEC (%) | | IEC (%) | | Non-Reduci ng CE-SDS (%) | Protein Content |
|---|---|---|---|---|---|---|---|---|
| | | | Mono mer | Polym er | Acid Peak | Neutr al peak | | |
| Stainle ss steel | 2-8°C | 0 | 99.46 | 0.09 | 20.77 | 61.82 | 94.70 | 52.27 |
| | | 8h | 99.42 | 0.08 | 20.69 | 62.02 | 96.17 | 51.10 |
| | | 24h | 99.75 | 0.00 | 20.64 | 62.21 | 95.03 | 52.89 |
| Liquid storage bag | 2-8°C | 0 | 99.46 | 0.09 | 20.77 | 61.82 | 94.70 | 52.27 |
| | | 8h | 99.47 | 0.14 | 20.67 | 61.94 | 96.19 | 51.15 |
| | | 24h | 99.76 | 0.02 | 20.75 | 61.89 | 96.12 | 52.41 |
| Glass bottle | 2-8°C | 0 | 99.46 | 0.09 | 20.77 | 61.82 | 94.70 | 52.27 |
| | | 8h | 99.72 | 0.06 | 20.54 | 62.24 | 96.30 | 51.02 |
| | | 24h | 99.62 | 0.00 | 20.71 | 61.95 | 96.39 | 51.14 |

### Example 13. Determination of compatibility of formulation with different filters

H001-4-YTE was prepared at 50 mg/mL in a solution comprising 10 mM histidine hydrochloride buffer, pH 6.0, with 25 mg/ml sucrose and 0.2 mg/ml polysorbate 80. The formulation sample was passed through 0.22 µm PES filters and PVDF filters, and samples were taken at 30 min and 1 h for testing. Analysis of protein content, appearance and purity indicated that H001-4-YTE was stable within 1 hour of contact with the filter. The formulation is compatible with both PES and PVDF filters.

**Table 14. Stability of H001-4-YTE in different materials of filter membranes**

| Material s of filter membra nes | Tim e | Appeara nce | SEC (%) | | IEC (%) | | Non-reduci ng CE-SDS (%) | Protein Content |
|---|---|---|---|---|---|---|---|---|
| | | | Monomer | Fragme nt | Aci d Pea k | Neutr al Peak | | |
| PES | 0h | Clear and transpare nt | 99.30 | 0.69 | 20.1 0 | 63.60 | 95.77 | 53.15 |
| | 0.5 h | Clear and transpare nt | 99.34 | 0.66 | 19.9 0 | 63.60 | 96.09 | 53.97 |
| | 1h | Clear and transpare nt | 99.00 | 1.00 | 20.3 0 | 63.30 | 96.03 | 53.11 |
| PVDF | 0h | Clear and transpare nt | 99.11 | 0.89 | 20.1 0 | 63.60 | 95.84 | 52.23 |
| | 0.5 h | Clear and transpare nt | 99.27 | 0.73 | 20.00 | 63.90 | 96.07 | 53.48 |
| | 1h | Clear and transpare nt | 99.21 | 0.79 | 20.0 0 | 63.90 | 96.02 | 52.42 |

### Example 14. Other alternative formulations of formulation

The pharmaceutical composition of the present invention can be used as a drug substance or an injection solution of a pharmaceutical formulation directly. When the pharmaceutical composition of the present invention is used as a stock solution of a pharmaceutical formulation, a lyophilized formulation is prepared by a lyophilization process and reconstituted into an injection solution for clinical use. The lyophilized formulation of the invention is prepared according to a process of lyophilization in low concentration and reconstitution in high concentration, that is, the low concentration of the pharmaceutical formulation solution is lyophilized to obtain a lyophilized formulation, and the lyophilized formulation is reconstituted into a higher concentration of pharmaceutical composition for clinical use. The lyophilized formulation has a longer stability than the liquid formulation. For the drug substance of pharmaceutical formulation used for lyophilized formulation, the higher the concentration of each component is, the longer the lyophilization time required. When various indicators setting of the lyophilization process are considered comprehensively, the concentration of each component of the injection after reconstitution of the pharmaceutical formulation is usually 2-5 times of the concentration of each component of the injection after reconstitution, and 3 times in the preferred embodiment of the present invention.

The stable pharmaceutical formulation provided by the present invention comprises: an anti-PCSK-9 antibody protein (non-limiting embodiment such as h001-4-YTE) and a combination of any of the following stable buffers:
(1) Antibody h001-4-YTE 150mg/ml, 75 mg/ml sucrose, 0.6 mg/ml polysorbate 80, and 30 mM histidine-hydrochloride buffer, pH 6.4;
(2) Antibody h001-4-YTE 150 mg/ml, 75 mg/ml sucrose, 0.6 mg/ml polysorbate 80, and 30 mM histidine-hydrochloride buffer, pH 6.2;
(3) Antibody h001-4-YTE 150mg/ml, 75 mg/ml sucrose, 0.6 mg/ml polysorbate 80, and 30 mM histidine-hydrochloride buffer, pH 6.3;
(4) Antibody h001-4-YTE 50mg/ml, 25 mg/ml sucrose, 0.2 mg/ml polysorbate 80, and 10 mM histidine-hydrochloride buffer, pH 6.4;
(5) Antibody h001-4-YTE 50mg/ml, 25 mg/ml sucrose, 0.2 mg/ml polysorbate 80, and 10 mM histidine-hydrochloride buffer, pH 6.3;
(6) Antibody h001-4-YTE 50 mg/ml, 25 mg/ml sucrose, 0.2 mg/ml polysorbate 80, and 10 mM histidine-hydrochloride buffer, pH 6.2;
(7) Antibody h001-4-YTE 50 mg/ml, 25 mg/ml sucrose, 0.2 mg/ml polysorbate 80, and 10 mM histidine-hydrochloride buffer, final pH 6.1;
(8) Antibody h001-4-YTE 50 mg/ml, 25 mg/ml sucrose, 0.2 mg/ml polysorbate 80, and 10 mM histidine-hydrochloride buffer, pH 6.0;
(9) Antibody h001-4-YTE 50 mg/ml, 25 mg/ml sucrose, 0.2 mg/ml polysorbate 80, and 10 mM histidine-hydrochloride buffer, pH 6.5;
(10) Antibody h001-4 50mg/ml, 25 mg/ml sucrose, 0.2 mg/ml polysorbate 80, and 10 mM histidine-hydrochloride buffer, pH 6.3;
(11) Antibody h001-4 50 mg/ml, 25 mg/ml sucrose, 0.2 mg/ml polysorbate 80, and 10 mM histidine-hydrochloride buffer, pH 6.2;
(12) Antibody h001-4 50 mg/ml, 25 mg/ml sucrose, 0.2 mg/ml polysorbate 80, and 10 mM histidine-hydrochloride buffer, pH 6.4;
(13) Antibody h001-4-YTE 50 mg/ml, 25 mg/ml sucrose, 0.1 mg/ml polysorbate 80, and 20 mM histidine-hydrochloride buffer, pH 6.3;
(14) Antibody h001-4-YTE 50 mg/ml, 25 mg/ml sucrose, 0.2 mg/ml polysorbate 80, and 15 mM histidine-hydrochloride buffer, pH 6.2;
(15) Antibody h001-4-YTE 50 mg/ml, 25 mg/ml sucrose, 0.2 mg/ml polysorbate 80, and 20 mM histidine-hydrochloride buffer, pH 6.4.
(16) Antibody h001-4-YTE 30 mg/ml, 10 mg/ml sucrose, 0.05 mg/ml polysorbate 80, and 5 mM histidine-hydrochloride buffer at pH 5.5;
(17) Antibody h001-4-YTE 70 mg/ml, 75 mg/ml sucrose, 0.6 mg/ml polysorbate 80, and 30 mM histidine-hydrochloride buffer at pH 6.5;
(18) Antibody h001-4-YTE 45 mg/ml, 20 mg/ml sucrose, 0.1 mg/ml polysorbate 80, and 8 mM histidine-hydrochloride buffer at pH 6.0;
(19) Antibody h001-4-YTE 55 mg/ml, 40 mg/ml sucrose, 0.3 mg/ml polysorbate 80, and 15 mM histidine-hydrochloride buffer at pH 6.2.

The pharmaceutical composition of the present invention can be prepared into a corresponding lyophilized formulation by a lyophilization process, and the lyophilized formulation can be reconstituted with an injection water to obtain the following pharmaceutical composition for clinical use:
(1) Anti-PCSK-9 antibody protein 150 mg/ml, 75 mg/ml sucrose, 0.6 mg/ml polysorbate 80, and 20 mM histidine-hydrochloride buffer, pH 6.3 ± 0.1;
(2) Anti-PCSK-9 antibody protein 120 mg/ml, 55 mg/ml sucrose, 0.4 mg/ml polysorbate 80, and 10 mM histidine-hydrochloride buffer, pH 6.0;
(3) Anti-PCSK-9 antibody protein 200 mg/ml, 95 mg/ml sucrose, 0.8 mg/ml polysorbate 80, and 30 mM histidine-hydrochloride buffer, pH 6.5.

Although the specific embodiments of the present invention have been described above, those skilled in the art will understand that these are merely illustrative. Many changes or modifications may be made to these embodiments without departing from the principle and essence of the invention. Therefore, the scope of the invention is defined by the appended claims.

## Claims

1. A pharmaceutical composition, comprising an anti-PCSK-9 antibody or an antigen-binding fragment thereof and a buffer, wherein the buffer is preferably a histidine buffer or succinate buffer or phosphate buffer or citrate buffer, more preferably a histidine buffer, most preferably a histidine-hydrochloride buffer.

2. The pharmaceutical composition according to claim 1, wherein the concentration of the anti-PCSK-9 antibody or the antigen-binding fragment thereof is about 1 mg/ml to 150 mg/ml, preferably about 30 mg/ml to 100 mg/ml, more preferably 45 mg/ml to 55 mg/ml, most preferably 50 mg/ml.

3. The pharmaceutical composition according to claim 1 or claim 2, wherein the pH of the pharmaceutical composition is about 5.5 to 6.5, preferably about 6.0 to 6.5, most preferably 6.0.

4. The pharmaceutical composition according to anyone of claims 1 to 3, wherein the concentration of the buffer is about 5 mM to 30 mM, preferably 5 mM to 20 mM, further preferably 5 mM to 15 mM, most preferably 10 mM.

5. The pharmaceutical composition according to anyone of claims 1 to 4, further comprises a disaccharide, wherein the disaccharide is preferably selected from trehalose or sucrose, most preferably sucrose.

6. The pharmaceutical composition according to claim 5, wherein the concentration of the disaccharide is about 10 mg/ml to 75 mg/ml, preferably about 20 mg/ml to 40 mg/ml, most preferably 25 mg/ml.

7. The pharmaceutical composition according to anyone claims of 1 to 6, further comprises a surfactant, wherein the surfactant is preferably polysorbate, more preferably polysorbate 80.

8. The pharmaceutical composition according to claim 7, wherein the concentration of the surfactant is about 0.05 mg/ml to 0.6 mg/ml, preferably 0.1 mg/ml to 0.4 mg/ml, further preferably 0.1 mg/ml to 0.3 mg/ml, most preferably 0.2 mg/ml.

9. The pharmaceutical composition according to anyone of claims 1 to 8, wherein the anti-PCSK-9 antibody or the antigen binding fragment thereof comprises HCDR1, HCDR2 and HCDR3 having the amino acid sequences of SEQ ID NO: 12, SEQ ID NO: 13 and SEQ ID NO: 14, respectively, and LCDR1, LCDR2 and LCDR3 having the amino acid sequences of SEQ ID NO: 15, SEQ ID NO: 16, and SEQ ID NO: 17, respectively.

10. The pharmaceutical composition according to claim 9, wherein the anti-PCSK-9 antibody or the antigen-binding fragment thereof comprises a heavy chain variable region selected from the group consisting of SEQ ID NOs: 18-23, and a light chain variable region selected from the group consisting of SEQ ID NOs: 24-27.

11. The pharmaceutical composition according to anyone of claims 1 to 10, wherein the light chain amino acid sequence of the anti-PCSK-9 antibody has at least 95% sequence identity to the antibody light chain amino acid sequence of SEQ ID NO: 30, the heavy chain amino acid sequence of the anti-PCSK-9 antibody has at least 95% sequence identity to the antibody heavy chain amino acid sequence of SEQ ID NO: 28 or 32.

12. A method for preparing the pharmaceutical composition according to anyone of claims 1 to 11, comprising a step of replacing the stock solution of an anti-PCSK-9 antibody or an antigen-binding fragment thereof with a buffer, wherein the buffer is preferably histidine buffer, wherein the concentration of the buffer is preferably about 5 mM to 30 mM, wherein the pH of the buffer is about 6.0 to 6.5.

13. The method according to claim 12, further comprising the step of adding sucrose and polysorbate 80 to the solution obtained from the step of replacing and then making up to a final volume with buffer, wherein the concentration of the buffer is preferably about 10 mM to 20 mM, wherein the pH of the buffer is about 6.0 to 6.5.

14. A method for preparing a lyophilized formulation comprising an anti-PCSK-9 antibody, which comprises the step of freeze-drying the pharmaceutical composition according to any one of claims 1 to 11.

15. The method for preparing a lyophilized formulation comprising the anti-PCSK-9 antibody according to claim 14, wherein the freeze-drying sequentially comprises the steps of pre-freezing, primary drying and secondary drying.

16. A lyophilized formulation comprising the anti-PCSK-9 antibody prepared by the method of any one of claims 14 to 15.

17. A method for preparing a reconstituted solution comprising an anti-PCSK-9 antibody, comprising the step of reconstituting the lyophilized formulation of claim 16, wherein the solvent for reconstitution is preferably water for injection.

18. A reconstituted solution comprising the anti-PCSK-9 antibody prepared by the method of claim 17.

19. The reconstituted solution comprising the anti-PCSK-9 antibody according to claim 18, wherein the concentration of the anti-PCSK9 antibody or the antigen binding fragment is about 120 mg/ml to 200 mg/ml, most preferably 150 mg/ml.

20. The reconstituted solution comprising the anti-PCSK-9 antibody according to claim 18, wherein the pH of the pharmaceutical composition is about 6.0-6.5, preferably 6.3.

21. The reconstituted solution comprising the anti-PCSK-9 antibody according to claim 18, wherein the concentration of the buffer is about 15 mM to 45 mM, preferably 30 mM.

22. The reconstituted solution comprising the anti-PCSK-9 antibody according to claim 18, further comprises a disaccharide, wherein the disaccharide is preferably selected from trehalose or sucrose, most preferably sucrose.

23. The reconstituted solution comprising the anti-PCSK-9 antibody according to claim 22, wherein the concentration of the disaccharide is about 55 mg/ml to 95 mg/ml, preferably 75 mg/ml.

24. The reconstituted solution comprising the anti-PCSK-9 antibody according to claim 18, further comprises a surfactant, wherein the surfactant is preferably polysorbate, more preferably polysorbate 80.

25. The reconstituted solution comprising the anti-PCSK-9 antibody according to claim 24, wherein the concentration of the surfactant is about 0.4 mg/ml to 0.8 mg/ml, preferably 0.6 mg/ml.

26. The use of the pharmaceutical composition according to anyone of claims 1 to 11, or the lyophilized formulation according to claim 16, or the reconstituted solution of the lyophilized formulation according to anyone claims of 18 to 25, in the formulation of a medicament for treatment of a PCSK9-mediated disease or disorder, wherein the disease or disorder is preferably a cholesterol-related disease; more preferably is selected from the group consisting of hypercholesterolemia, heart disease, metabolic syndrome, diabetes, coronary heart disease, apoplexy, cardiovascular disease, Alzheimer's disease and general Dyslipidemia; most preferably hypercholesterolemia, dyslipidemia, atherosclerosis, CVD or coronary heart disease.

27. A method of treating and preventing PCSK-9-related disease or disorder comprising administering to a patient in need thereof a therapeutically effective amount of the pharmaceutical composition of anyone of claims 1 to 11 or the lyophilized formulation according to claim 16 or the reconstituted solution of the lyophilized formulation according to anyone claims of 18 to 25, wherein the disease or disorder is preferably a cholesterol-related disease; more preferably is selected from the group consisting of hypercholesterolemia, heart disease, metabolic syndrome, diabetes, coronary heart disease, apoplexy, cardiovascular disease, Alzheimer's disease and general Dyslipidemia; most preferably hypercholesterolemia, dyslipidemia, atherosclerosis, CVD or coronary heart disease.

28. Aproduct, comprising a container containing the pharmaceutical composition of anyone of claims 1 to 11 or the lyophilized formulation of claim 16 or the reconstituted solution of the lyophilized formulation according to anyone of claims of 18 to 25.
